# EUROPEAN PATENT APPLICATION

(11) **EP 4 527 379 A1**
(43) Date of publication of application: **26.03.2025**
(21) Application number: 23198201.8
(22) Date of filing: 19.09.2023
(51) Int. Cl.: A61K 9/51, A61K 9/127, A61K 9/00

(54) **LIPIDS CONTAINING AN OLIGOGLYCEROL GROUP**

(71) Applicant: Thermosome GmbH, 82152 Planegg (DE)
(72) Inventor: HOSSANN, Martin., 82152 Planegg (DE); PETRINI, Matteo., 82152 Planegg (DE); MAYER, Susanne., 82152 Planegg (DE); PETROV, Orlin., 82152 Planegg (DE); MICHAELIS, Uwe., 82152 Planegg (DE); LINDNER, Lars., 81377 München (DE)
(74) Representative: Weickmann & Weickmann PartmbB

(57) **Abstract**

The present invention relates to oligoglycerol-containing lipids and in particular to cationic or cationizable oligoglycerol-containing lipids and to a method for providing these oligoglycerol-containing lipids. Further, the present invention relates to liposomes, in particular thermosensitive liposomes comprising the novel oligoglycerol-containing lipids. Further the invention relates to liposomes comprising the novel oligoglycerol-containing lipids, which liposomes have an adjustable surface charge.

## Description

The present invention relates to oligoglycerol-containing lipids and in particular to cationic or cationizable oligoglycerol-containing lipids and to a method for providing these oligoglycerol-containing lipids. Further, the present invention relates to liposomes, in particular thermosensitive liposomes comprising the novel oligoglycerol-containing lipids. Further the invention relates to liposomes comprising the novel oligoglycerol-containing lipids, which liposomes have an adjustable surface charge.

Liposomes are spherical vesicles formed by a membrane bilayer composed by lipid excipients and are widely used and accepted as drug delivery nanocarriers. Today, many natural and synthetic lipids are well known excipients in liposomal formulations.

Thermosensitive liposomes (TSL) are improved liposomal drug delivery systems allowing active targeting of tumor tissue and of other diseased areas, such as inflamed tissue or joints, via heat-induced local release of the encapsulated active pharmaceutical ingredients (APIs).

Most patients with advanced tumor diseases die from progressive tumor growth despite the successes achieved with classic chemotherapy and cancer immunotherapy. Efficient drug targeting is therefore a key issue in medical research (Lindner & Hossann, 2010). Classic small-molecule API lack intrinsic affinity for diseased tissue, and considerable quantities of them are distributed throughout healthy tissue after systemic application. For API, such as those used in cancer therapy, this systemic availability is associated with severe side effects. Therefore, effective dosing of these API is limited, and the therapeutic potential of many conventional non-targeted drugs cannot be fully exploited.

With the help of innovative drug delivery systems (DDS) for targeted drug delivery, the efficiency of known drugs can be improved and the risk of occurring drug resistance can be reduced. Of the DDS developed to date, liposomes and lipid-based nanoparticles (LNPs) have achieved the greatest relevance for clinical use. Liposomes are versatile and often classified as biocompatible DDS. The pharmacodynamics of liposomally encapsulated API is determined by the lipid excipient composition, the route of administration (e.g., intravenous, intratumoral., oral), the distribution of the liposomes, the type of targeting (e.g., passive, active, targeted) and the rate at which the drug is released from the liposomes.

Therefore, novel innovative DDS are needed showing affinity for the diseased tissue and limited distribution to healthy tissue.

For example, PEGylated long-circulating formulations like Caelyx^{®} have been designed to exploit the enhanced permeability and retention (EPR) effect and passively accumulate inside tumor tissue because of the leaky tumor vasculature. Accumulation of nanoparticle in tumor tissue depends on the tumor environment, a natural barrier for DDS (Nakamura et al. 2016). Extravasation of the vesicles is the rate-limiting step and nanoparticles must circulate for days to accumulate in sufficient high concentrations. Because accumulation in tumor tissue competes with uptake by the mononuclear phagocyte system (MPS) in liver and spleen only less than 5% of injected dose accumulate in tumor (O'Brien et al. 2004). As result, Caelyx^{®} did not show improved efficacy compared to free drug (Harrington et al 2001) due to known shortcomings of the formulation.

Wilhelm et al. surveyed relevant literature from 2005 to 2015 and concluded that only a 0.7% (median) of the injected dose of miscellaneous DDS was delivered to solid tumors (Wilhelm et al. 2016). The review article was critically debated (Torrice 2016). It was stated that the U.S. Food and Drug Administration (FDA) and nanoparticle developers usually judge delivery systems by following the drug and not the nanocarrier by pharmacokinetic (PK) parameters. Another point of criticism was, that strong-performing systems may have been statistically lost among many weaker once and the efficacy of the nanoparticle system was not compared to free drug. Nevertheless, one could conclude, that efficacy of nanoparticle delivery via EPR effect was not significantly improved in one decade. Wilhelm et al. suggested that a first step to improve DDS would be to investigate how they leave tumor vessels and/or interact with tumor tissues. Recently, it was demonstrated that up to 97% of nanoparticles enter tumors rather by an active process through endothelial cells (Sindhwani et al. 2020). Such an active process might be steered by the ability of the DDS to interact with the responsible cells and by characteristics of the DDS (e.g., absence of PEGylation, lipid composition, composition of protein corona, surface charge, size).

Surface modification of liposomal DDS with anionic oligoglycerol-containing lipid excipients is known (e.g., WO 97/30058, WO 2014/202680). It was shown that the number of glycerol moieties in anionic 1,2-dipalmitoyl-*sn*-glycero-3-phospho-oligoglycerols (DPPGₓ) can steer the pharmacokinetic behavior and organ distribution of liposomes (WO 97/30058, Schagon 1997). A TSL formulation based on the anionic 1,2-dipalmitoyl-*sn*-glycero-3-phospho-diglycerol (DPPG₂) or 1,2-dipalmitoyl-*sn*-glycero-3-phospho-triglycerol (DPPG₃) encapsulating the drug doxorubicin (DOX) showed superiority over clinically relevant DOX formulations in treatment of sarcoma in a rat sarcoma model (Hossann et al. 2021). Liposomes containing DPPG₂ allowed a better interaction and/or uptake into cells when compared to PEGylated liposomes (Petrini et al. 2021). Furthermore, the content of DPPG₂ in a formulation notably influenced the composition of the protein corona and hemocompatibility of the liposomal DDS (Lokerse et al. 2021). DPPGₓ was used to replace PEGylated phospholipids in cholesterol-containing (WO 97/30058 A1) and cholesterol-free liposomes (Hossann et al. 2021) to prolong the circulation half-life of these nanocarriers. Alternatives like DPPGₓ or the cationic or cationizable oligoglycerol-containing lipids as described hereinafter are of interest as novel excipients for liposomal DDS because usage of PEGylation in human drug products was questioned in recent years for several reasons (Hoang et al. 2020).

An object of the present invention was therefore to provide novel oligoglycerol lipid excipients. These lipids should in particular allow for the formation of anionic, cationic, or cationizable liposomal DDS having an adjustable surface charge, that are covered by oligoglycerol moieties on their surface.

The present invention relates to an oligoglycerol-containing lipid of Formula (I) 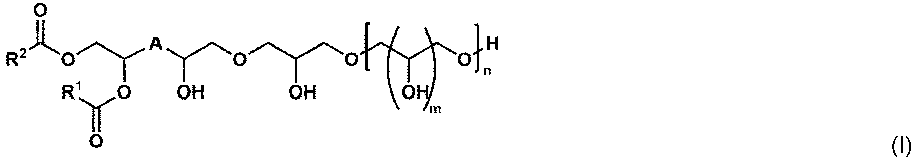 wherein
R¹ and R² each independently are a hydrocarbon group having 12 to 24 C-atoms; A = NR³ or N⁺R⁴R⁵ ;
R³, R⁴ and R⁵ each independently are hydrogen or a hydrocarbon group having 1 to 5 C-atoms;
n = a number from 0 to 20, and
m is 0 or 1.

The inventive oligogylcerol-containing lipids have at least two glycerol moieties. According to the invention n is a number from 0 to 20. Preferably, n is a number from 0 to 10, more preferably from 0 to 5. Most preferred n is 0, 1 or 2.

The index m in formula (I) can be, at each occurrence independently 0 or 1. For m=1 a glycerol group is indicated, for m=0 an ethylene glycol group is indicated.

In particular, n is 0, 1 or 2, and m = 1, such that the oligoglycerol-containing lipid of Formula (I) is a diglycerol (n=0), a triglycerol (n=1) or a tetraglycerol (n=3), respectively. Even more preferably, n is 0 or 1 and most preferably n is 0.

The inventive oligoglycerol-containing lipid contains a cationizable or cationic group A. When A = NR³ a cationizable oligoglycerol-containing lipid is indicated, which is also designated NLGₓ herein (x denotes the number of glycerol groups). When A = N⁺R⁴R⁵ a cationic oligoglycerol-containing lipid is indicated, which is also designated CLGₓ herein (x denotes the number of glycerol groups). Preferred is an oligoglycerol-containing lipid, wherein A = NR³ and R³ =CH₃ or wherein A = N⁺R⁴R⁵ and R⁴, R⁵ = CH₃.

In the oligoglycerol-containing lipids of Formula (I), residues R¹ and R² each independently are a hydrocarbon group having 12 to 24 C-atoms. Preferred is an oligoglycerol-containing lipid, wherein R¹ and R² independently are a hydrocarbon group having 13 to 19 C-atoms. The hydrocarbon groups can be saturated or mono- or polyunsaturated. The hydrocarbon groups can be linear or branched. Preferably R¹ and R² independently are a linear or branched saturated C13- to C19-alkyl group, more preferably a linear saturated C13- to C19-alkyl group. More preferred R¹ and R² independently are a linear saturated C15 or C17 alkyl group, most preferred a linear saturated C15 alkyl group.

Most preferred are NLG₂ having formula (III) 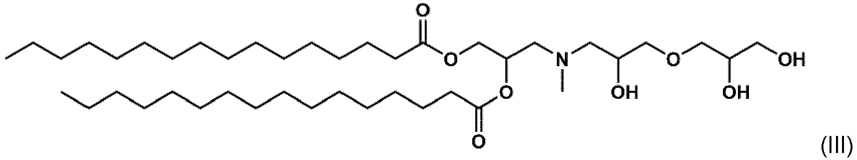 and CLG₂ having formula (IV).

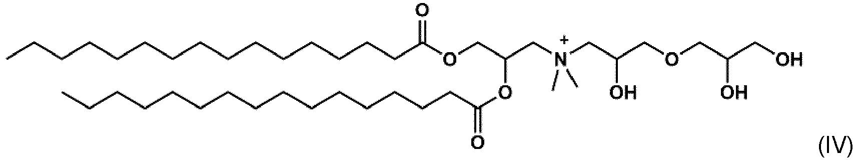

The inventors of the present application succeeded in providing the oligoglycerol-containing lipid of Formula (I) in a stereoisomeric form. Therefor the invention further relates to a stereoisomeric oligoglycerol-containing lipid having Formula (II) 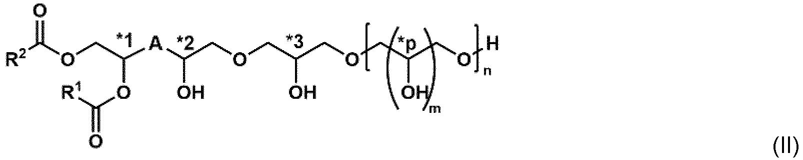 wherein the three stereogenic centers *1, *2 and *3 have a predetermined configuration and in particular wherein stereogenic center *1 is in R-configuration, stereogenic center *2 is in S-configuration and stereogenic center *3 is in R-configuration; and wherein *p denotes further stereogenic centers. The further stereogenic centers independently may have a predetermined configuration, either R or S.

A suitable counter ion for cationic lipids is CI⁻ .

The invention further provides a method for preparing an oligoglycerol-containing lipid having Formula (I) as herein described. The method is characterized in that the synthesis route includes a central intermediate of Formula (V) or of Formula (V') 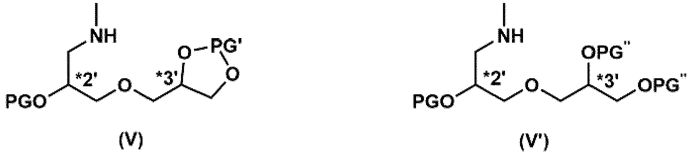 wherein PG, PG' and PG" independently are protecting groups. PG and PG" in particular are benzyl (Bn), tetrahydropyranyl (THP), ethoxyethyl (EE), 2-methoxypropan-2-yl (MOP), silyl or para-methoxybenzyl (PMB).For the synthesis of an oligoglycerol-containing lipid of Formula (I), wherein R¹ and R² independently are saturated hydrocarbon group preferably PG and PG" is benzyl (Bn). For the synthesis of an oligoglycerol-containing lipid of Formula (I), wherein R¹ and R² independently are a mono- or polyunsaturated hydrocarbon group preferably PG and PG" is THP and silyl. PG' in particular is CH₂ (methylene), CHCH₃ (ethylidene), CHPh (benzylidene), CHPh*p*-Ome (para-methoxybenzylidene), C(CH₃)₂ (isopropylidene) or Si*t*Bu₂ (di-tert-butylsilylene), Si(iPr)₂-O-Si(iPr)₂ (tetraisopropyldisiloxanylidene), preferably PG' is C(CH₃)₂.

Preferably the two stereogenic centers *2' and *3' have a predetermined configuration. It was found that a synthesis route including a central intermediate of Formula (V) or (V'), wherein the two stereogenic centers *2' and *3' have a predetermined configuration allows for preparation of oligoglycerol-containing lipids of Formula (II), wherein the three stereogenic centers *1, *2 and *3 can be provided with a predetermined configuration.

For obtaining higher oligoglycerols, in particular tri- or tetraglycerols, the intermediate of Formula (V) or (V') is repeatedly reacted with a glycidol to form building blocks having an increased number of glycerols:

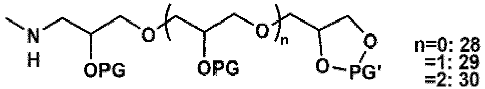

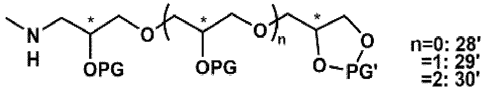

A synthesis overview is provided in the scheme below showing a method which is in particular suitable for the synthesis of cationizable or cationic lipids wherein R¹ and R² are saturated alkyl groups. PG and PG" independently are protecting groups. The depicted synthesis proceeds in a stereospecific manner and not only stereoselective. With the use of starting materials being single stereoisomers or enantiomers the product obtained is a oligoglycerol-containing lipid being a single stereoisomer of Formula (II). Exemplarily the synthesis of diglycerols is shown.

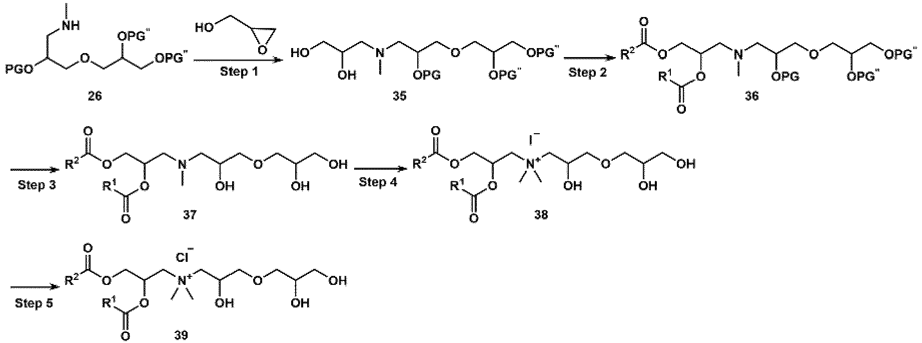

A further synthesis overview is provided in the scheme below showing a method which is in particular suitable for the synthesis of cationizable or cationic lipids wherein R¹ and R² are unsaturated alkyl groups. PG and PG' independently are protecting groups. The depicted synthesis proceeds in a stereospecific manner and not only stereoselective. With the use of starting materials being single stereoisomers or enantiomers the product obtained is a oligoglycerol-containing lipid being a single stereoisomer of Formula (II). Exemplarily the synthesis of diglycerols, triglycerols and tetraglycerols is shown.

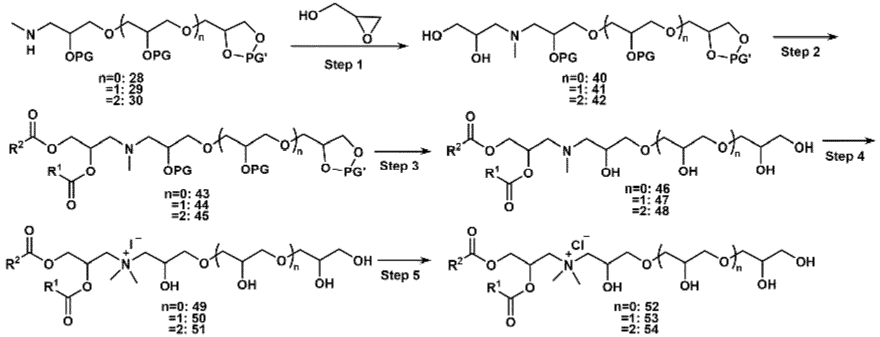

A further synthesis overview is provided in the scheme below showing a method which is in particular suitable for the synthesis of anioniclipids wherein R¹ and R² are unsaturated or saturated alkyl groups. PG, PG' and PG² independently are protecting groups. PG² in particular is acetyl (Ac), chloroacetyl, dichloroacetyl, trichloroacetyl and phenoxyacetyl. The depicted synthesis proceeds in a stereospecific manner and not only stereoselective. With the use of starting materials being single stereoisomers or enantiomers the product obtained is a oligoglycerol-containing lipid being a single stereoisomer of Formula (II). Exemplarily the synthesis of diglycerols, triglycerols and tetraglycerols is shown.

As reagents for phosphorylation phosphoramidite chlorides or phosphordiamidites are used, wherein R' denotes a leaving group LG. R' in particular is 2-cyanoethyl (CNE). R" is a C1-C12 hydrocarbon group, in particular R" is methyl (Me), ethyl (Et), isopropyl (iPr) or benzyl (Bn), preferably R" is isopropyl.

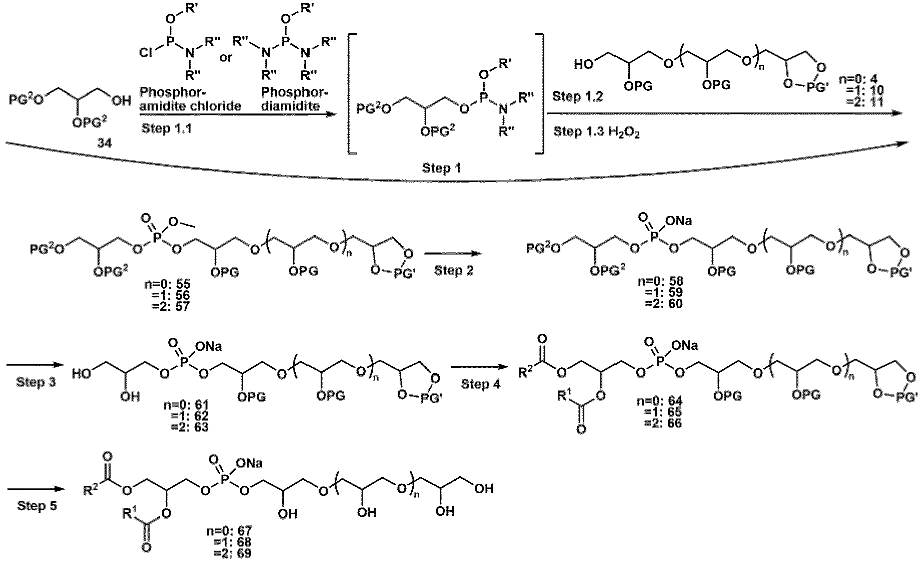

Synthesis of oligoglycerol building blocks in general is shown in the following schemes, wherein PG, PG' and PG' independently are protecting groups. PG, PG' and PG' may be orthogonal protecting groups. The depicted synthesis proceeds in a stereospecific manner and not only stereoselective. With the use of starting materials being single stereoisomers or enantiomers the product obtained is a oligogylcerol building block being a single stereoisomer of Formula (V) or (V') or of Formula (VI').

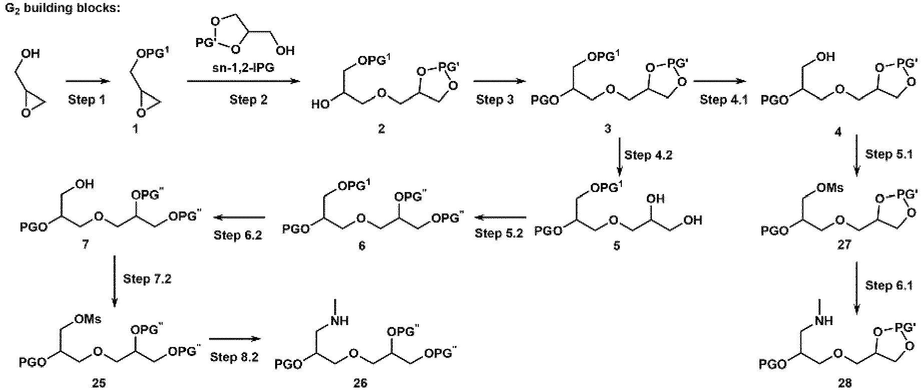

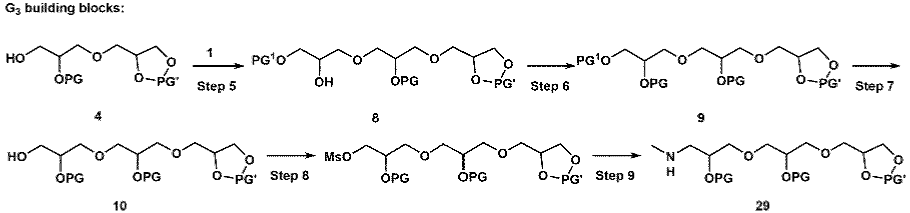

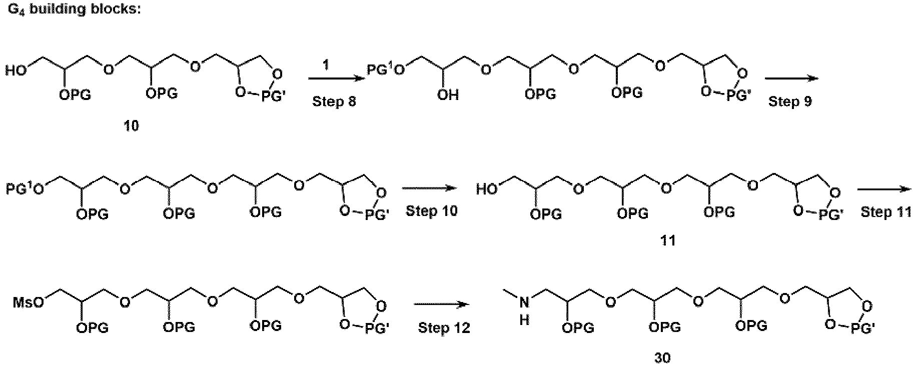

A preferred method using sn-1,2-diacyl-glycerol for providing lipid building blocks for unsaturated lipid is shown in the following, wherein PG' and PG² independently are protecting groups. PG² in particular is acetyl (Ac), chloroacetyl, dichloroacetyl, trichloroacetyl and phenoxyacetyl.

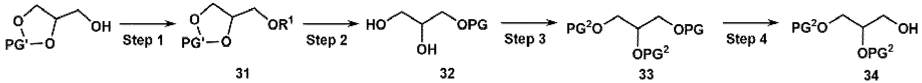

The oligoglycerol-containing lipid of the present invention is in particular suitable for preparing liposomes. Therefor the present invention also relates to a liposome comprising an oligoglycerol-containing lipid of the invention.

The cationic surface charge of drug delivery systems DDS (e.g., liposomes, LNP) is conventionally achieved by mixing neutral glycerophospholipid excipients and/or cholesterol with cationic (e.g., 1,2-dioleoyl-3-trimethylammonium-propane, DOTAP) or ionizable lipid-like compounds (e.g., 1,2-dioleoyl-3-dimethylammonium-propane, DODAP). According to the invention a cationic surface charge is provided by oligoglycerol-modified cationic or cationizable lipids and in particular by diglycerol-modified cationic or cationizable lipids. The aim of the present invention was to provide a novel class of cationizable or cationic lipid-based excipients (AXGₓ). The novel lipids either carry a permanent or a pH-dependent variable positive charge and an oligoglycerol in the polar head group, respectively.

The cationizable NLG₂ of formula (III) 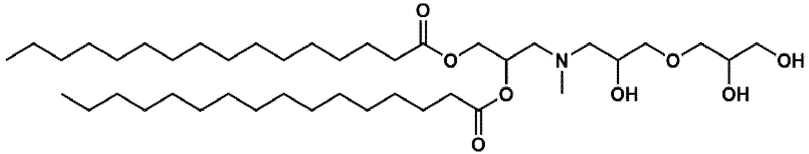 and the cationic CLG₂ of formula (IV) 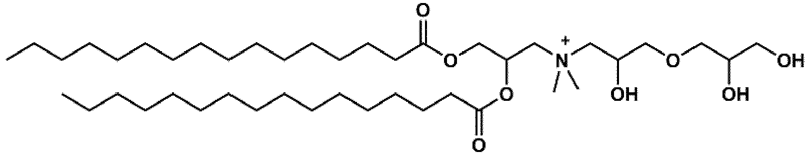 are preferred lipids from the AXGₓ class. Stearic acid or palmitic acid as esterified fatty acid and a diglycerol moiety as headgroup are most preferred. NLG₂ and CLG₂ are a cationizable and cationic analogue to the anionic glycerophospholipid DPPG₂ (1,2-dipalmitoyl-*sn-*glycero-3-phospho-diglycerol).

The present invention further relates to a liposome comprising at least one oligoglycerol-containing lipid of Formula (I).

The inventive liposome in particular comprises at least one oligoglycerol-containing lipid of Formula (I) 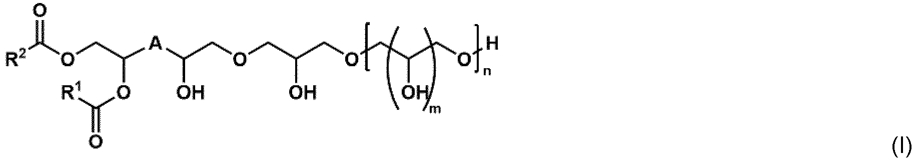 wherein R¹ and R² each independently are a hydrocarbon group having 12 to 24 C-atoms;
A = NR³ or N⁺R⁴R⁵;
R³, R⁴ and R⁵ each independently are hydrogen or a hydrocarbon group having 1 to 5 C-atoms;
n = a number from 0 to 20, and
m is 0 or 1.

It has been found that the inventive cationizable or cationic lipids are perfectly suitable lipid excipients for liposomes. By the inventive lipids a positive charge can be conferred to the liposomes.

In a preferred embodiment the inventive liposome of the invention comprises at least one oligoglycerol-containing lipid of Formula (I) 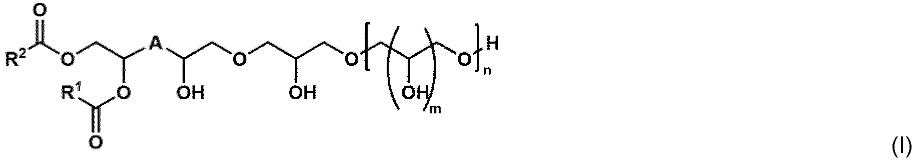 wherein R¹ and R² each independently are a hydrocarbon group having 12 to 24 C-atoms;
A = NR³;
R³ is hydrogen or a hydrocarbon group having 1 to 5 C-atoms;
n = a number from 0 to 20, and
m is 0 or 1 and
at least one phosphatidyloligoglycerol of Formula (VI) 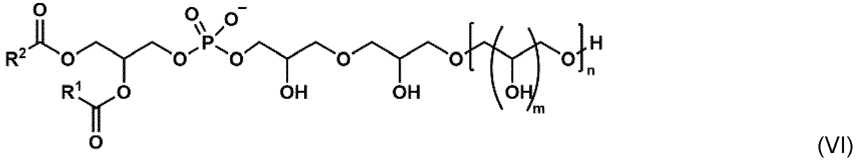
wherein R¹ and R² each independently are a hydrocarbon group having 12 to 24 C-atoms and wherein n is a number from 0 to 20, and m is 0 or 1.

Preferred meanings of R¹ and R², n and m are as given herein above. The phosphatidyloligoglycerol of Formula (VI) is also termed DPPGₓ herein.

Preferably the phosphatidyloligoglycerol is of Formula (VI') 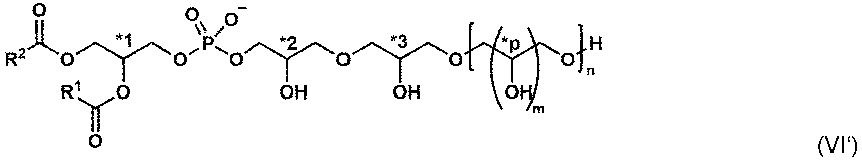
wherein the three stereogenic centers *1, *2 and *3 have a predetermined configuration; and *p denotes further stereogenic centers; and
wherein R¹ and R² each independently are a hydrocarbon group having 12 to 24 C-atoms; n is a number from 0 to 20; and
m at each occurrence independently is 0 or 1 and in particular
wherein stereogenic center *1 is in R-configuration, stereogenic center *2 is in S-configuration and stereogenic center *3 is in R-configuration and *p denotes further stereogenic centers.

Most preferably the phosphatidyloligoglycerol has Formula (VI")

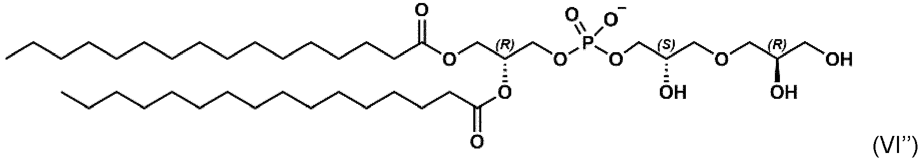

The phosphatidyloligoglycerol of Formula (VI") is also termed DPPG₂ herein.

In a further preferred embodiment the liposome of the invention comprises at least one oligoglycerol-containing lipid of Formula (I) 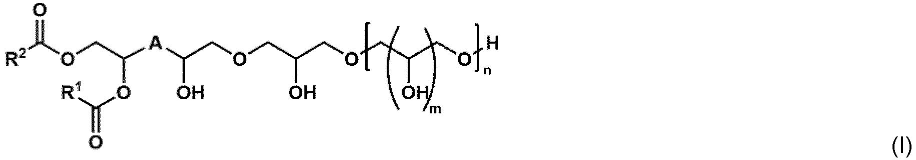 wherein R¹ and R² each independently are a hydrocarbon group having 12 to 24 C-atoms;
A = N⁺R⁴R⁵;
R⁴ and R⁵ each independently are hydrogen or a hydrocarbon group having 1 to 5 C-atoms;
n = a number from 0 to 20, and
m is 0 or 1; and
at least one oligoglycerol-containing lipid of Formula (I) 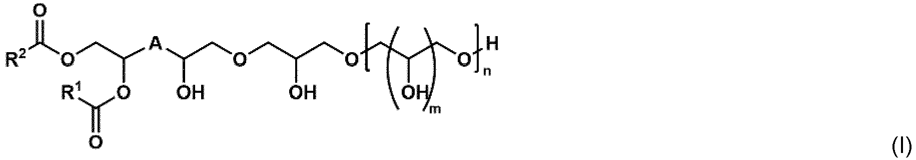
wherein R¹ and R² each independently are a hydrocarbon group having 12 to 24 C-atoms;
A = NR³;
R³ is hydrogen or a hydrocarbon group having 1 to 5 C-atoms;
n = a number from 0 to 20, and
m is 0 or 1.

Preferred inventive liposomes comprise at least one cationizable oligoglycerol-containing lipid (A=NR³) and at least one anionic phosphatidyloligoglycerol or at least one cationizable oligoglycerol-containing lipid (A=NR³) and at least one cationic oligoglycerol-containing lipid (A=N⁺R⁴R⁵). Such a preferred inventive liposome is a drug delivery system having oligoglycerol groups on its surface, while the surface charge is adjustable due to inclusion of cationic, cationizable and/or anionic groups in varying amounts. In particular, the zeta potential can be adjusted by the relative amounts of cationic and/or cationizable oligogylcerol-containing lipids of Formula (I) and of phosphatidyloligoglycerols of Formula (VI).

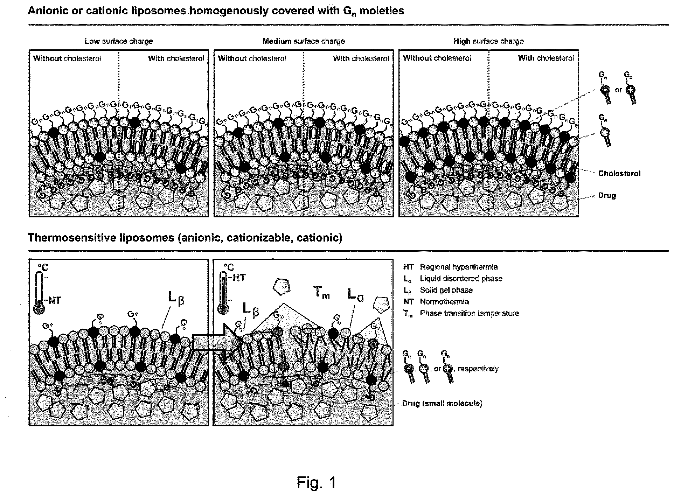

The application of the three oligoglycerol-containing lipid excipients allows the customdesign of liposomal DDS regarding the magnitute of their surface charge and the length of the oligoglycerol-chains covering the surface. For the first time, the surface charge (anionic or cationic) is independently adjustable by the ratio of the three types of excipients (of anionic, cationic, and cationizable) while the amount of oligoglycerol moieties on the DDS surface can be kept constant (Figure 1). Moreover, the intra- and extraliposomal surface charge is independently adjustable by the pH of the intra- and extraliposomal aqueous buffer. Finally, the liposomal DDS can be designed to disintegrate in acidic environments (e.g., tumor tissue, lysosomes) to release their payload.

In general, the membrane bilayer of liposomal formulations is composed of more than one lipid excipient, but not every combination of lipids can be mixed in liposomal formulations. The miscibility of the used lipids is essential to form homogeneous bilayer membranes and to ensure stability and functionality. In case of incompatibility of lipid excipients, the lipids segregate and phase separation or composition fluctuations will happen. Miscibility of phospholipids with identical headgroups but chain lengths differing by two methylene groups is dependent on headgroup structure and on headgroup charge. Therefore, miscibility might be remedied by adjusting the length and degree of saturation of the acyl/alkyl chains However, in individual cases, liposomal formulation can be designed to force lipid excipient de-mixing at defined conditions, e.g., heat-induced formation of lysolipid-assisted membrane pores in TSL.

It was found that oligoglycerol-containing lipids of Formula (I) form homogeneous bilayer membranes and to ensure stability and functionality of liposomes including these lipids. Besides miscibility of at least two oligoglycerol-containing lipids of Formula (I) surprisingly also miscibility of oligoglycerol-containing lipids of Formula (I) with phosphatidyloligoglycerols of Formula (VI) was found allowing to provide liposomes having a tailored surface charge.

The inventive liposomes may comprise further excipients.

In an embodiment the liposome according to the invention preferably comprises at least one phosphatidylcholine of formula (VII) 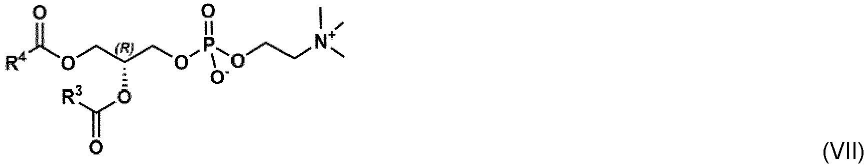 wherein R³ and R⁴ each independently represent a hydrocarbon group having from 12 to 24 carbon atoms. R³ and R⁴ are preferably each independently of the other a saturated or mono- or poly-unsaturated, preferably linear alkyl group, in particular a saturated linear alkyl group. R³ and R⁴ are further preferably each independently a C13 to C19, in particular a C15 to C17 hydrocarbon group. Preferably, R³ and R⁴ are each independently a linear saturated C13- to C23-, in particular a C13- to C21-alkyl group. Most preferably, R³ and R⁴ are independently a linear saturated C15- or C17-alkyl group. 1,2-dipalmitoyl-*sn*-glycero-3-phosphocholine (DPPC) and 1,2-distearoyl-sn-glycero-3-phosphocholine (DSPC) are particularly preferred.

The liposome, and in particular the thermosensitive liposome according to the invention, preferably comprises a phosphatidylcholine of formula (VII) in the natural configuration.

In a further embodiment the liposome comprises cholesterol.

In a preferred embodiment the inventive liposome is a thermosensitive liposome. Thermosensitive liposomes (TSL) are improved liposomal drug delivery systems allowing active targeting of tumor tissue via heat-induced local release of the encapsulated active pharmaceutical ingredients (APIs).

In preferred embodiments of the invention the liposome comprises (all mol.-% are based on the lipid content of the liposome):
(1) NLG_{X}, in particular NLG₂, preferably in an amount of at least 1 mol.%, more preferably at least 10 mol.-% ; and/or
(2) CLGₓ, in particular CLG₂, preferably in an amount of at least 1 mol.%, more preferably at least 10 mol.-% ; and/or
(3) NLG_{X}, in particular NLG₂, and CLGₓ, in particular CLG₂, preferably in an amount of at least 1 mol.% each, more preferably at least 10 mol.-% each; and/or
(4) NLG_{X}, in particular NLG₂, preferably in an amount of at least 1 mol.%, more preferably at least 10 mol.-% ; and at least one phosphatidylcholine, preferably in an amount of at least 1 mol.-%, preferably at least 10 mol.-%; and/or
(5) NLG_{X}, in particular NLG₂, preferably in an amount of at least 1 mol.%, more preferably at least 5 mol.-% and up to 80 mol.-%, more preferably up to 75 mol.-% ; and DPPC, preferably in an amount of at least 1 mol.%, more preferably at least 5 mol.-% and up to 80 mol.-%, more preferably up to 75 mol.-%; and DSPC, preferably in an amount of at least 1 mol.%, more preferably at least 10 mol.-% and up to 30 mol.-%, more preferably up to 25 mol.-%; and/or
(6) NLG_{X}, in particular NLG₂, preferably in an amount of at least 1 mol.%, more preferably at least 5 mol.-% and up to 50 mol.-%, more preferably up to 40 mol.-%; and at least one phosphatidylcholine, preferably DSPC and/or DPPC, preferably in an amount of at least 1 mol.-%, preferably at least 20 mol.-% .-% and up to 70 mol.-%, more preferably up to 60 mol.-%; and cholesterol, preferably in an amount of at least 1 mol.-%, preferably at least 30 mol.-% and up to 70 mol.-%, more preferably up to 50 mol.-%; and/or
(7) CLGₓ, in particular CLG₂, preferably in an amount of at least 1 mol.%, more preferably at least 10 mol.-% ; and at least one phosphatidylcholine, preferably in an amount of at least 1 mol.-%, preferably at least 10 mol.-%; and/or
(8) CLGₓ, in particular CLG₂, preferably in an amount of at least 1 mol.%, more preferably at least 5 mol.-% and up to 80 mol.-%, more preferably up to 75 mol.-% ; and DPPC, preferably in an amount of at least 1 mol.%, more preferably at least 5 mol.-% and up to 80 mol.-%, more preferably up to 75 mol.-%; and DSPC, preferably in an amount of at least 1 mol.%, more preferably at least 10 mol.-% and up to 30 mol.-%, more preferably up to 25 mol.-%; and/or
(9) CLGₓ, in particular CLG₂, preferably in an amount of at least 1 mol.%, more preferably at least 5 mol.-% and up to 50 mol.-%, more preferably up to 40 mol.-%; and at least one phosphatidylcholine, preferably DSPC and/or DPPC, preferably in an amount of at least 1 mol.-%, preferably at least 20 mol.-% .-% and up to 70 mol.-%, more preferably up to 60 mol.-%; and cholesterol, preferably in an amount of at least 1 mol.-%, preferably at least 30 mol.-% and up to 70 mol.-%, more preferably up to 50 mol.-%; and/or
(10) NLG_{X}, in particular NLG₂, and CLGₓ, in particular CLG₂, preferably in an amount of at least 1 mol.% each, preferably at least 10 mol.-% each; and cholesterol, preferably in an amount of at least 1 mol.-%, preferably at least 30 mol.-% and up to 70 mol.-%, more preferably up to 50 mol.-%; and/or
(11) NLG_{X}, in particular NLG₂, and CLGₓ, in particular CLG₂, preferably in an amount of at least 1 mol.% each, preferably at least 10 mol.-% each; and at least one phosphatidylcholine, preferably in an amount of at least 1 mol.-%, preferably at least 10 mol.-%; and/or
(12) NLG_{X}, in particular NLG₂, and CLGₓ, in particular CLG₂, preferably in an amount of at least 1 mol.% each, preferably at least 10 mol.-% each; and at least one phosphatidylcholine, preferably DSPC and/or DPPC, preferably in an amount of at least 1 mol.-%, preferably at least 20 mol.-% .-% and up to 70 mol.-%, more preferably up to 60 mol.-% and cholesterol, preferably in an amount of at least 1 mol.-%, preferably at least 30 mol.-% and up to 70 mol.-%, more preferably up to 50 mol.-%; and/or
(13) NLG_{X}, in particular NLG₂, preferably in an amount of at least 1 mol.%, more preferably at least 5 mol.-% and up to 80 mol.-%, more preferably up to 75 mol.-% ; and CLGₓ, in particular CLG₂, preferably in an amount of at least 1 mol.%, more preferably at least 5 mol.-% and up to 80 mol.-%, more preferably up to 75 mol.-%; and DPPC, preferably in an amount of at least 1 mol.%, more preferably at least 5 mol.-% and up to 80 mol.-%, more preferably up to 75 mol.-%; and DSPC, preferably in an amount of at least 1 mol.%, more preferably at least 10 mol.-% and up to 30 mol.-%, more preferably up to 25 mol.-%; and/or
(14) NLG_{X}, in particular NLG₂, preferably in an amount of at least 1 mol.%, more preferably at least 5 mol.-% and up to 99 mol.-%, more preferably up to 95 mol.-% ; and DPPGₓ, in particular DPPG₂, preferably in an amount of at least 1 mol.%, more preferably at least 5 mol.-% and up to 99 mol.-%, more preferably up to 95 mol.-%;
(15) NLG_{X}, in particular NLG₂, and DPPGₓ, in particular DPPG₂, preferably in an amount of at least 1 mol.% each, preferably at least 10 mol.-% each; and cholesterol, preferably in an amount of at least 1 mol.-%, preferably at least 30 mol.-% and up to 70 mol.-%, more preferably up to 50 mol.-%;
(16) NLG_{X}, in particular NLG₂, and DPPGₓ, in particular DPPG₂, preferably in an amount of at least 1 mol.% each, preferably at least 10 mol.-% each; and at least one phosphatidylcholine, preferably in an amount of at least 1 mol.-%, preferably at least 10 mol.-%;
(17) NLGₓ, in particular NLG₂, and DPPGₓ, in particular DPPG₂, preferably in an amount of at least 1 mol.% each, preferably at least 10 mol.-% each; and at least one phosphatidylcholine, preferably DSPC and/or DPPC, preferably in an amount of at least 1 mol.-%, preferably at least 20 mol.-% .-% and up to 70 mol.-%, more preferably up to 60 mol.-% and cholesterol, preferably in an amount of at least 1 mol.-%, preferably at least 30 mol.-% and up to 70 mol.-%, more preferably up to 50 mol.-%; and/or
(18) NLG_{X}, in particular NLG₂, preferably in an amount of at least 1 mol.%, more preferably at least 5 mol.-% and up to 80 mol.-%, more preferably up to 75 mol.-% ; and DPPGₓ , in particular DPPG₂, preferably in an amount of at least 1 mol.%, more preferably at least 5 mol.-% and up to 80 mol.-%, more preferably up to 75 mol.-%; and DPPC, preferably in an amount of at least 1 mol.%, more preferably at least 5 mol.-% and up to 80 mol.-%, more preferably up to 75 mol.-%; and DSPC, preferably in an amount of at least 1 mol.%, more preferably at least 10 mol.-% and up to 30 mol.-%, more preferably up to 25 mol.-%.

By setting the amounts and proportions respectively of CLGₓ, NLGₓ and DPPGₓ the surface charge of the inventive liposomes can be adjusted.

The inventive liposome further preferably comprises an active agent, in particular a cytostatic drug. Most preferred the inventive liposome comprises doxorubicin (DOX) which is a cytotoxic anthracycline. Further suitable cytostatic anthracyclines presently employed in cancer treatment include, epirubicin, idarubicin, daunorubicin and mitoxantrone. In further embodiments of the invention other cytostatics such as mitomycin C, gemcitabine, trabectedin, etc. or platinum derivatives such as cisplatin, carboplatin or oxaliplatin can be included in the liposome.

In a further preferred embodiment a liposome according to the invention, comprises an active pharmaceutical ingredient which is selected from the group consisting of anthracyclines such as doxorubicin, daunorubicin, idarubicin, epirubicin aclarubicin, amrubicin, pirarubicin, valrubicin and zorubicin; anthracenediones such as mitoxantrone and pixantrone; antineoplastic antibiotics such as mitomycin and bleomycin; vinca alkaloids such as vinblastine, vincristine and vinorelbine; alkylating agents such as cyclophosphamide and mechlorethamine hydrochloride: campthothecins such as topotecan, irinotecan (CPT-1 1), lurtotecan, 9-aminocamptothecin, 9-nitrocamptothecin and 10-hydroxycamptothecin; purine and pyrimidine derivatives such as 5-fluorouracil, gemcitabine (2,2'-difluoro-2-deoxycytidine, dFdC), floxuridine (FUDR), cytarabine (cytosine arabinoside), 6-azauracil (6-AU); oxazaphosphorines such as cyclophosphamide, ifosfamide and trofosfamide; taxanes such as paclitaxel and docetaxel; podophyllotoxin derivatives such as etopside and teniposide; platinum-based compounds such as cisplatin, carboplatin, oxaliplatin, nedaplatin; methotrexate; tyrosine kinase inhibitors such as imatinib, gefitinib, erlotinib, sunitinib, adavosertib and lapatinib; and cytarabines such as cytosine arabinoside.

### References

Carrasco MJ, Alishetty S, Alameh MG, Said H, Wright L, Paige M, Soliman O, Weissman D, Cleveland TE 4th, Grishaev A, Buschmann MD. Ionization and structural properties of mRNA lipid nanoparticles influence expression in intramuscular and intravascular administration. Commun Biol. 2021;4(1):956. DOI: 10.1038/s42003-021-02441-2
Chonn A, Cullis PR, Devine DV. The role of surface charge in the activation of the classical and alternative pathways of complement by liposomes. J Immunol 1991;146:4234-41. DOI: 10.3109/08982109209010213
Dézsi L, Fülöp T, Mészáros T, Szénási G, Urbanics R, Vázsonyi C, Őrfi E, Rosivall L, Nemes R, Kok RJ, Metselaar JM, Storm G, Szebeni J. Features of complement activationrelated pseudoallergy to liposomes with different surface charge and PEGylation: comparison of the porcine and rat responses. J Control Release. 2014;195:2-10. DOI: 10.1016/j.jconrel.2014.08.009
Harrington KJ, Mohammadtaghi S, Uster PS, Glass D, Peters AM, Vile RG, Stewart JS. Effective targeting of solid tumors in patients with locally advanced cancers by radiolabeled pegylated liposomes. Clin Cancer Res. 2001;7(2):243-54. DOI: not available
Hoang Thi TT, Pilkington EH, Nguyen DH, Lee JS, Park KD, Truong NP. The importance of poly(ethylene glycol) alternatives for overcoming PEG immunogenicity in drug delivery and bioconjugation. Polymers 2020;12:298. DOI: 10.3390/polym12020298
Hossann M, Wiggenhorn M, Schwerdt A, Wachholz K, Teichert N, Eibl H, Issels RD, Lindner LH. In vitro stability and content release properties of phosphatidylglyceroglycerol containing thermosensitive liposomes. Biochim Biophys Acta. 2007;1768(10):2491-9. DOI: 10.1016/j.bbamem.2007.05.021
Hossann M, Wang T, Wiggenhorn M, Schmidt R, Zengerle A, Winter G, Eibl H, Peller M, Reiser M, Issels RD, Lindner LH. Size of thermosensitive liposomes influences content release. J Control Release. 2010;147(3):436-43. DOI: 10.1016/j.jconrel.2010.08.013 Hossann M, Syunyaeva Z, Schmidt R, Zengerle A, Eibl H, Issels RD, Lindner LH. Proteins and cholesterol lipid vesicles are mediators of drug release from thermosensitive liposomes. J Control Release. 2012;162(2):400-6. DOI: 10.1016/j.jconrel.2012.06.032
Hossann M, Hirschberger J, Schmidt R, Baumgartner C, Zimmermann K, Baer S, et al. A heat-activated drug delivery platform based on phosphatidyl-(oligo)-glycerol nanocarrier for effective cancer treatment. Adv NanoBiomed Res 2021;1:2000089. DOI: 10.1002/anbr.202000089
Javanainen M, Martinez-Seara H, Vattulainen I. Nanoscale membrane domain formation driven by cholesterol. Sci Rep. 2017 Apr 25;7(1):1143. DOI: 10.1038/s41598-017-01247-9
Knudsen KB, Northeved H, Kumar PE, Permin A, Gjetting T, Andresen TL, Larsen S, Wegener KM, Lykkesfeldt J, Jantzen K, Loft S, Møller P, Roursgaard M. In vivo toxicity of cationic micelles and liposomes. Nanomedicine. 2015;11(2):467-77. DOI: 10.1016/j.nano.2014.08.004
Lasic DD, Woodle MC, Papahadjopoulos D. On the molecular mechanism of steric stabilization of liposomes in biological fluids. J Liposome Res 1992;2:335-53. DOI: 10.3109/08982109209010213
Lindner LH, Hossann M. Factors affecting drug release from liposomes. Curr Opin Drug Discov Devel. 2010 Jan;13(1):111-23. DOI: not available
Lokerse WJM, Lazarian A, Kleinhempel A, Petrini M, Schwarz P, Hossann M, Holdt LM, Mailänder V, Lindner LH. Mechanistic investigation of thermosensitive liposome immunogenicity and understanding the drivers for circulation half-life: A polyethylene glycol versus 1,2-dipalmitoyl-sn-glycero-3-phosphodiglycerol study. J Control Release. 2021;333:1-15. DOI: 10.1016/j.jconrel.2021.03.014
Nakamura Y, Mochida A, Choyke PL, Kobayashi H. Nanodrug delivery: is the enhanced permeability and retention effect sufficient for curing cancer? Bioconjug Chem. 2016;27(10):2225-2238. DOI: 10.1021/acs.bioconjchem.6b00437
O'Brien ME, Wigler N, Inbar M, Rosso R, Grischke E, Santoro A, Catane R, Kieback DG, Tomczak P, Ackland SP, Orlandi F, Mellars L, Alland L, Tendler C; Reduced cardiotoxicity and comparable efficacy in a phase III trial of liposomal doxorubicin HCl (CAELYX/Doxil) versus conventional doxorubicin for first-line treatment of metastatic breast cancer. Ann Oncol. 2004;15(3):440-9. DOI: 10.1093/annonc/mdh097
Schagon O. Liposomen als potentielle Arzneistoffträger: Variation der biopharmazeutischen Eigenschaften durch 1,2-Dipalmitoyl-sn-glycero-oligo-glycerine. Shaker Verlag, Aachen; 1997. ISBN: 978-3-8265-2479-0
Sindhwani S, Syed AM, Ngai J, Kingston BR, Maiorino L, Rothschild J, MacMillan P, Zhang Y, Rajesh NU, Hoang T, Wu JLY, Wilhelm S, Zilman A, Gadde S, Sulaiman A, Ouyang B, Lin Z, Wang L, Egeblad M, Chan WCW. The entry of nanoparticles into solid tumours. Nat Mater. 2020;19(5):566-575. DOI: 10.1038/s41563-019-0566-2.
Szebeni J, Bedocs P, Rozsnyay Z, Weiszhár Z, Urbanics R, Rosivall L, Cohen R, Garbuzenko O, Báthori G, Tóth M, Bünger R, Barenholz Y. Liposome-induced complement activation and related cardiopulmonary distress in pigs: factors promoting reactogenicity of Doxil and AmBisome. Nanomedicine. 2012;8(2):176-84. DOI: 10.1016/j.nano.2011.06.003.
Petrini M, Lokerse WJM, Mach A, Hossann M, Merkel OM, Lindner LH. Effects of surface charge, PEGylation and functionalization with dipalmitoylphosphatidyldiglycerol on liposome-cell interactions and local drug delivery to solid tumors via thermosensitive liposomes. Int J Nanomedicine. 2021;16:4045-4061. DOI: 10.2147/IJN.S305106.
Torrice M. Does Nanomedicine Have a Delivery Problem? ACS Cent Sci. 2016;2(7):434-7. DOI: 10.1021/acscentsci.6b00190
Wilhelm S, Tavares AJ, Dai Q, Ohta S, Audet J, Dvorak HF, et al. Analysis of nanoparticle delivery to tumours. Nature Reviews Materials 2016;1:16014EP. DOI: 10.1038/natrevmats.2016.14

The appended figures further illustrate the invention.
Figure 1: Application of novel anionic, cationic, and/or cationizable lipid excipient in liposomal formulations.
Figure 2: Dispersion stability (DLS) of binary mixtures of NLG₂, CLG₂ and DPPG₂. Dotted red lines marks quality criteria for size and PDI. Red squares indicate PDI values OOS. Numbers below the bars indicate extrusion pore size. Hydration solution.
Figure 3: Dispersion stability (DLS) of ternary mixtures of HSPC/Chol/AXGₓ. Z-average and PDI of (A) HSPC/Chol/CLG₂ 60-x:40:x (mol/mol) and (B) HSPC/Chol/NLG₂ 60-x:40:x (mol/mol) batches. Zeta potential of (C) HSPC/Chol/CLG₂ 60-x:40:x (mol/mol) and (D) HSPC/Chol/NLG₂ 60-x:40:x (mol/mol) batches. (x = 5 to 40). The dotted red lines mark the quality criteria for z average and PDI. Numbers below graphs (A) and (B) indicate the used extrusion membrane pore size during batch production.
Figure 4: Dispersion stability (DLS) of ternary mixtures of CLG₂/NLG₂/Chol. Investigated formulations: CLG₂/NLG₂/Chol 50:10:40 (mol/mol) and CLG₂/NLG₂/Chol 30:30:40 (mol/mol). (A) Z-average and PDI (after preparation), and (B) zeta potential. The dotted red lines mark the quality criteria for z average and PDI. Numbers below graphs indicate the used extrusion membrane pore size during batch production.
Figure 5: Effect of NLG₂ content in TSL on the solid gel to liquid disordered phase transition temperature (Tₘ). Refer to Table 1 for the corresponding peak temperatures of the heating and cooling phase.
Figure 6: Effect of extraliposomal pH on zeta potential of DPPC/DSPC/NLG₂ liposomes. The assay was performed with (A) DPPC/DSPC/NLG₂ 75/20/5 (mol/mol) liposomes, (B) DPPC/DSPC/NLG₂ 70/20/10 (mol/mol) liposomes, (C) DPPC/DSPC/NLG₂ 60/20/20 (mol/mol) liposomes, and (D) DPPC/DSPC/NLG₂ 50/20/30 (mol/mol) liposomes.
Figure 7: Effect of extraliposomal pH on z average & PDI of DPPC/DSPC/NLG₂ liposomes. The assay was performed with (A) DPPC/DSPC/NLG₂ 75/20/5 (mol/mol) liposomes, (B) DPPC/DSPC/NLG₂ 70/20/10 (mol/mol) liposomes, (C) DPPC/DSPC/NLG₂ 60/20/20 (mol/mol) liposomes, and (D) DPPC/DSPC/NLG₂ 50/20/30 (mol/mol) liposomes. Mean and S.D. of three experiments.
Figure 8: *In vitro* complement activation of AXGₓ-based liposomes. ELISA on *in vitro* complement activation of different liposomal formulations in human plasma. SC5b-9 was used as read-out. Zymosan: positive control. Buffer: negative control. Red dotted line indicates the SC5b-9 value induced by the control.
Figure 9: Binding of cationizable DPPC/DSPC/NLG₂ liposomes to B16 cells. Live cell fluorescence microscopy: bright field (top) and RGB merged pictures (bottom). Scale bar = 50 µm.
Figure 10: Binding of neutral and anionic liposomes to B16 cells. Live cell fluorescence microscopy: bright field (top) and RGB merged pictures (bottom). Scale bar = 50 µm.
Figure 11: CLG₂-based TSL showed agglutination of THP-1 cells. (A) Cells treated with DPPC/DSPC/CLG₂ 70:20:10 (mol/mol) (refer also to Table 2, #6). (B) Control.
Figure 12: Vesicle size and zeta potential of DPPC/DSPC/NLG₂ TSL loaded with CF. Selected DLS results: (A) Vesicle size (z-average size) and PDI. The dotted line marks vesicle size and PDI acceptance criteria. (B) Zeta potential. N=3 for batches extruded with 100 or 50 nm filters. N=1 for batches extruded with 200 nm filters.
Figure 13: Effect of vesicle size and NLG₂ content on the temperature-dependent drug release (TDR) profile of DPPC/DSPC/NLG₂ TSL. The dotted line marks 50% CF-release.
Figure 14: Effect of CLG₂ content on the temperature-dependent drug release (TDR) profile of DPPC/DSPC/CLG₂ TSL. Temperature dependent CF release was measured at distinct temperatures for an incubation time of (A) 5 minutes and (B) 60 minutes. Red dashed line indicates peak release within mild-HT temperature range (41-43 °C).
Figure 15: Effect of DOX:lipid ratio on heat-triggered drug release from DPPC/DSPC/CLG₂ TSL. (A,C) Temperature-dependent drug release (TDR) profile for DPPC/DSPC/CLG₂ 75:20:5 (mol/mol), (B,D) TDR profile for DPPC/DSPC/CLG₂ 70:20:10 (mol/mol).

### Examples

### Example 1: Synthesis route of saturated cationic/cationizable lipids

The synthesis route for new lipids NLG₂ and CLG₂ is depicted below. The synthesis proceeds via a central intermediate of formula (VI'), in particular 26.1a. NLG₂ is obtained in 11 synthesis steps in total and 3 steps from 26.1a. The cationic CLG₂ was obtained in 2 additional steps from NLG₂. The synthesis route is applicable to other AXGₓ lipids. The central intermediate is benzyl-protected (26.1a) and the fatty acid chloride is fully saturated and does not contain double bonds, respectively.

### Synthesis route of NLG₂ and CLG₂

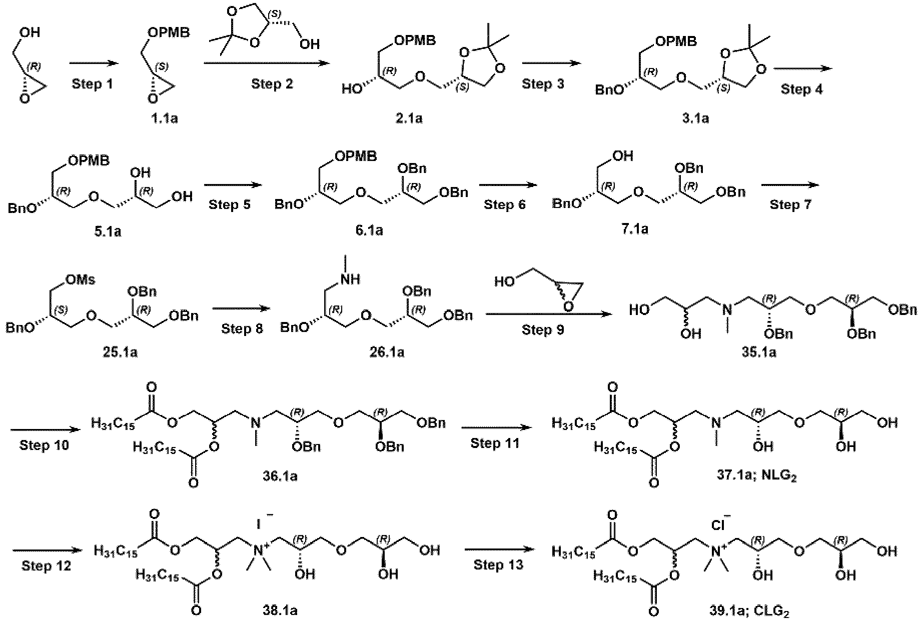

### Step 1:

3.8 kg (R)-(+)-glycidol are dissolved in 22.8 L NMP, then 8.02 kg PMBCI are added followed by 2.26 kg NaH at 0-5 °C. After 4 hours at RT, the reaction mixture is poured into ice water and extracted with EtOAc. The organic phase is washed with brine, dried over Na₂SO₄ and concentrated under reduced pressure. The isolated residue is purified by silica gel chromatography (n-Heptane/Ethyl acetate) and 8.0 kg of **1.1a** are obtained as colorless oil. It is characterized by HPLC, TLC and ¹H-NMR.

¹H NMR of **1.1a** (400 MHz, CDCl₃):
δ ppm 7.29 (d, *J* = 10.8 Hz, 2H), 6.89 (d, *J* = 10.8 Hz, 2H), 4.53 (q, *J* = 11.6 Hz, 2H), 3.79 (s, 3H), 3.74 (dd, *J* = 3.2, 11.6 Hz, 1H), 3.41 (dd, *J* = 6.0, 11.6 Hz, 1H), 3.17 (m, 1H), 2.79 (dd, *J* = 4.0, 9.2 Hz, 1H), 2.61 (dd, *J* = 2.4, 4.8 Hz, 1H).

### Step 2+3:

8.0 kg of **1.1a** are dissolved in 64 L DMF, then 8.16 kg sn-1,2-IPG is added, followed by 6.94 kg tBuOK at 0-5 °C and the resulting reaction mixture stirred for 12 h at RT to obtain **2.1a**. Characterization is done with TLC and HPLC. Subsequently, without work up or further purification additional 6.91 kg tBuOK and 12.6 kg benzyl bromide are added at 0-5 °C and the resulting reaction mixture stirred at 4 h at RT. Then, the reaction mixture is poured into ice water and extracted with EtOAc. The organic phase is washed with brine, dried over Na₂SO₄ and concentrated under reduced pressure. 7.0 kg of pure compound **3.1a** is obtained as yellow oil after purification by silica gel chromatography (n-Heptane/Ethyl acetate). **3.1a** is characterized with TLC, HPLC, and ¹H-NMR.

¹H NMR of **3.1a** (400 MHz, CDCl₃):
δ ppm 7.39 - 7.26 (m, 8H), 6.92 (d, *J* = 8.4 Hz, 2H), 4.71 (s, 2H), 4.50 (s, 2H), 4.27 (d, *J* = 6.0 Hz, 1H), 4.10 - 4.02 (m, 1H), 3.85 (s, 3H), 3.83 - 3.47 (m, 9H), 1.44 (s, 3H), 1.39 (s, 3H).

### Step 4:

379 g **3.1a** are dissolved in 2.65 L MeOH and then cooled to 0 °C, where 2 L 1M HCI are slowly added. The reaction mixture is stirred at 25 °C for 4 h. Then, saturated NaHCOs solution is added to adjust the mixture to pH = 7 at 0 - 5 °C, followed by extracted with EtOAc. The organic phase is washed brine, dried over Na₂SO₄, filtered and concentrated under reduced pressure to obtain 310 g of crude **5.1a** as yellow oil.

### Step 5:

310 g of **5.1a** are dissolved in 2.5 L DMF and then cooled to 0 °C, where 369 g tBuOK and 422 g BnBr are added. The reaction mixture is stirred at 25 °C for 4 h, before being poured into ice water followed by extraction with EtOAc. The organic phase is washed brine, dried over Na₂SO₄, filtered and concentrated under reduced pressure. After silica column chromatography (n-heptane/EtOAc) 115 g of pure **6.1a** are obtained as yellow oil.

¹H NMR of **6.1a** (400 MHz, CDCl₃):
δ ppm 7.32 - 7.10 (m, 17H), 6.81 - 6.74 (m, 2H), 4.60 (d, *J* = 4.0 Hz, 4H), 4.45 (s, 2H), 4.37 (s, 2H), 3.71 (s, 3H), 3.70 - 3.65 (m, 2H), 3.55 - 3.44 (m, 8H).

### Step 6:

331 g of **6.1a** are dissolved in 3 L ACN and 332 ml H₂O and then cooled to 0 °C, where 652 g cerium ammonium nitrate (CAN) are added. The reaction mixture is stirred at 15 °C for 8 h, before being poured into ice water followed by extraction with EtOAc. The organic phase is washed brine, dried over Na₂SO₄, filtered and concentrated under reduced pressure. After silica column chromatography (n-heptane/EtOAc) 168 g of pure **7.1a** are obtained as yellow oil.

¹H NMR of **7.1a** (400 MHz, CDCl₃):
δ ppm 7.32 - 7.10 (m, 17H), 6.81 - 6.74 (m, 2H), 4.60 (d, *J* = 4.0 Hz, 4H), 4.45 (s, 2H), 4.37 (s, 2H), 3.71 (s, 3H), 3.70 - 3.65 (m, 2H), 3.55 - 3.44 (m, 8H).

### Step 7:

153 g **7.1a** are dissolved in 765 ml DCM, then 71 g triethylamine added, followed by dropwise addition of 50.1 g MsCl. The reaction mixture is stirred at 20 °C for 2 h, before being poured into water followed by extraction with DCM. The organic phase is dried over Na₂SO₄, filtered and concentrated under reduced pressure to obtain 183 g of crude **25.1a** as yellow oil.

### Step 8:

183 g **25.1a** are dissolved in 366 ml EtOH and the resulting solution is added to 1.48 kg of a MeNH₂ solution (33% wt) in EtOH. The reaction mixture is warmed to 55°C and stirred for 48 h, before evaporation to dryness. The residue is taken up in MeOH/toluene and washed with 10 % aqueous Na₂CO₃ solution, before concentration under reduced pressure to obtain 151 g of crude **26.1a** as yellow oil.

¹H NMR of **26.1a** (400 Hz, CDCl₃):
δ ppm 7.30 - 7.18 (m, 16H), 4.65 - 4.61 (m, 3H), 4.51 - 4.46 (m, 3H), 3.69 (td, *J* = 5.2, 8.0 Hz, 2H), 3.57 - 3.45 (m, 6H), 2.61 (d, *J* = 5.6 Hz, 2H), 2.30 (s, 3H).

### Step 9:

43.6 g **26.1a** are dissolved in 109 ml toluene and then a solution of 9 g glycidol in 109 ml toluene added. The reaction mixture is warmed to 100°C and stirred for 8 h. 3 batches are run in parallel, then combined and evaporated to dryness. The residue is taken up in MTBE and adjusted to pH5 with HCI and then phases separated. The aqueous phase is adjusted to pH8 with saturated NaHCO₃ solution and extracted with ethyl acetate, before concentration of the organic phase under reduced pressure to obtain 161 g of crude **35.1a** as yellow oil.

### Step 10:

161 g **35.1a** are dissolved in 800 ml DCM and then 62.2 g triethylamine, 11.2 g DMAP and 169 g palmitoyl chloride added subsequently. The resulting reaction mixture is stirred at 20°C for 1 h, before pouring into water. After separation the organic phase is concentrated under reduced pressure and then purified by silica column chromatography (n-heptane/EtOAc) to obtain 146 g of pure **36.1a** as yellow oil.

¹H NMR of **36.1a** (400 Hz, CDCl₃):
δ 7.36 - 7.15 (s, 16H), 5.20 - 5.13 (m, 1H), 4.72 - 4.53 (m, 6H), 4.35 (dd, *J* = 3.2, 12.0 Hz, 1H), 4.10 - 4.04 (m, 1H), 3.79 - 3.70 (m, 1H), 3.68 - 3.51 (m, 8H), 2.61 - 2.51 (m, 4H), 2.31 - 2.24 (m, 7H), 2.06 (s, 1H), 1.64 - 1.55 (m, 6H), 1.26 (br s, 53H), 0.89 (t, *J* = 7.2 Hz, 6H).

### Step 11:

6 g Pd/C is suspended in 300 ml AcOH and then 67 g of **36.1a** are added and the reactor flushed with 30 psi H₂. The resulting reaction mixture is stirred at 30°C for 24 h. 2 batches are run in parallel and worked up together. After filtration through celite, the filtrate is poured into water and the pH adjusted to 7 with saturated NaHCO₃ solution. After extraction with ethyl acetate, the organic phase is concentrated under reduced pressure. The residue is recrystallized from ACN, followed by purification via silica column chromatography (n-heptane/EtOAc/MeOH) to obtain 38.6 g of pure **NLG₂** as white solid.

¹H NMR of **NLG₂** (400 Hz, CDCl₃):
δ 5.22 (dd, *J* = 2.4, 4.8 Hz, 1H), 4.30 (dd, *J* = 3.2, 12.0 Hz, 1H), 4.08 (dd, *J* = 6.4, 12.0 Hz, 1H), 3.91 - 3.79 (m, 2H), 3.72 - 3.44 (m, 7H), 2.67 (dd, *J* = 7.6, 13.6 Hz, 1H), 2.56 - 2.47 (m, 2H), 2.43 - 2.37 (m, 1H), 2.35 - 2.27 (m, 7H), 1.65 - 1.56 (m, 4H), 1.35 - 1.20 (m, 52H), 0.87 (t, *J* = 7.2 Hz, 6H).

### Step 12:

20 g **NLG₂** are suspended in 100 ml ACN and then 7.8 g methyl iodide is added. The resulting reaction mixture is stirred at 40°C for 16 h. After filtration and concentration under reduced pressure 21 g of crude **38.1a** are obtained as white solid.

¹H NMR of **38.1a** (400 Hz, DMSO)
δ ppm 5.56 - 5.48 (m, 2H), 4.62 (d, *J* = 4.8 Hz, 1H), 4.50 (br t, *J* = 5.4 Hz, 1H), 4.39 (dd, *J* = 3.2, 12.0 Hz, 1H), 4.22 (br s, 1H), 4.05 (dd, *J* = 5.6, 12.0 Hz, 1H), 3.82 (br d, *J* = 3.6 Hz, 2H), 3.63 - 3.55 (m, 1H), 3.49 - 3.39 (m, 4H), 3.37 - 3.28 (m, 5H), 3.18 - 3.09 (m, 6H), 2.42 - 2.25 (m, 4H), 1.57 - 1.47 (m, 4H), 1.23 (s, 48H), 0.88 - 0.81 (m, 6H).

### Step 13:

21 g **38.1a** are added to a 315 ml 1:1:1 mixture of toluene, 2-propanol and a 10% aqueous NaCl solution. The resulting reaction mixture is stirred at 35°C for 48 h. After isolation, crude product is purified by recrystallization from 2-propanol, followed by multiple triturations with 2-propanol at RT for 1 h to obtain 10 g pure **CLG₂** as white solid. Characterization by ICP-MS confirms CLG₂ as chloride salt.

¹H NMR of **CLG₂** (400 Hz, DMSO):
δ 5.65 - 5.51 (m, 2H), 4.43 (br d, J = 2.0 Hz, 1H), 4.41 - 4.36 (m, 1H), 4.28 (br d, J = 4.4 Hz, 2H), 4.10 (br dd, J = 5.2, 12.0 Hz, 1H), 3.94 - 3.79 (m, 2H), 3.62 (br s, 1H), 3.54 - 3.34 (m, 9H), 3.19 (br d, J = 3.2 Hz, 6H), 2.39 - 2.28 (m, 4H), 1.56 (br d, J = 6.4 Hz, 4H), 1.26 (br s, 48H), 0.93 - 0.80 (m, 6H).

### Example 2: Synthesis route of unsaturated cationic/cationizable lipids

The synthesis route for unsaturated new lipids NLG₂ and CLG₂ is depicted below. The synthesis proceeds via a central intermediate of formula (VI), in particular 28.1a. NLG₂ is obtained in 9 synthesis steps in total and 3 steps from 28.1a. The cationic CLG₂ was obtained in 2 additional steps from NLG₂. The synthesis route is applicable to other AXGₓ lipids. The central intermediate is silyl- and acetonide-protected (28.1a) and the fatty acid chloride is monounsaturated.

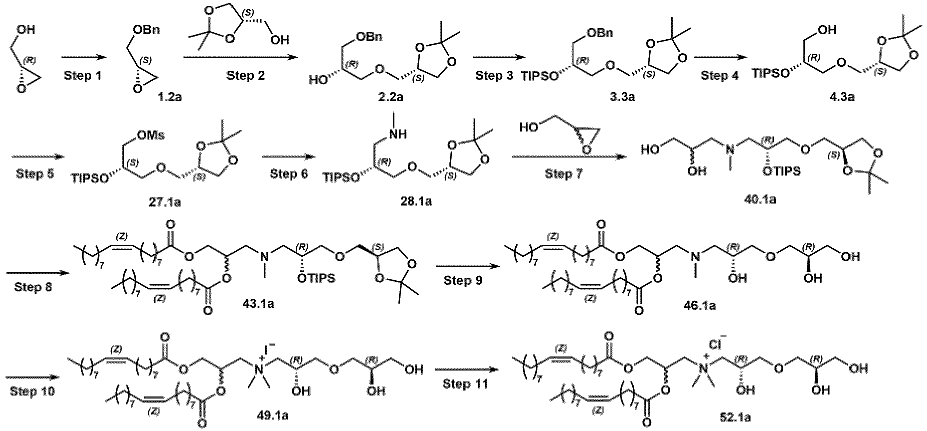

### Step 1:

(R)-(+)-glycidol is added to a suspension of NaH in DMF at 0 °C, followed by BnBr. The resulting reaction mixture is warmed to RT and stirred until completion of the reaction. Afterward, water is added, and the mixture extracted with DCM. The organic layer is washed with water and brine, dried over Na₂SO₄, filtered and concentrated under reduced pressure. The resulting residue is purified by silica gel chromatography (n-Heptane/Ethyl acetate) to obtain **1.2a**.

### Step 2:

**1.2a** is dissolved in DMF, then *sn*-1,2-IPG is added, followed by tBuOK at 0-5 °C and the resulting reaction mixture is stirred at RT until completion of the reaction. Then, the reaction mixture is poured into ice water and extracted with EtOAc. The organic phase is washed with brine, dried over Na₂SO₄ and concentrated under reduced pressure to obtain crude **2.2a**.

### Step 3:

Triisopropylsilyl chloride is added to a solution of **2.2a** and imidazole in DMF and the resulting reaction mixture is stirred at RT until completion of the reaction. Then, the reaction mixture is poured into water and extracted with EtOAc. The organic phase is washed with 10% citric acid and brine, then dried over Na₂SO₄ and concentrated under reduced pressure to obtain crude **3.3a**.

### Step 4:

**3.3a** is added to a suspension of Pd/C in EtOH/DCM, followed by degassing. The resulting mixture is stirred under H₂ (0.5 MPa) at RT until completion of the reaction. The mixture is filtered and the filtrate concentrated under reduced pressure. Crude **4.3a** is purified with silica gel chromatography (n-Heptane/Ethyl acetate).

### Step 5:

**4.2a** dissolved in DCM, then triethylamine added, followed by dropwise addition of MsCl. The reaction mixture is stirred at 20 °C until completion of the reaction. Then, the mixture is poured into water followed by extraction with DCM. The organic phase is dried over Na₂SO₄, filtered and concentrated under reduced pressure to obtain crude **27.1a.**

### Step 6:

**27.1a** is dissolved in EtOH and the resulting solution is added to a MeNH₂ solution (33% wt) in EtOH. The reaction mixture is warmed to 55°C and stirred until completion of the reaction, followed by evaporation to dryness. The residue is taken up in MeOH/toluene and washed with 10% aqueous Na2CO3 solution, before concentration under reduced pressure to obtain crude **28.1a**.

### Step 7:

**28.1a** is dissolved in toluene and then a solution of glycidol in toluene added. The reaction mixture is warmed to 100°C and stirred until completion of the reaction, followed by evaporation to dryness. The residue is taken up in MTBE and adjusted to pH5 with HCI and then phases separated. The aqueous phase is adjusted to pH8 with saturated NaHCOs solution and extracted with ethyl acetate, before concentration of the organic phase under reduced pressure to obtain crude **40.1a**.

### Step 8:

**40.1a** is dissolved in DCM and then triethylamine, DMAP and oleyl chloride added subsequently. The resulting reaction mixture is stirred at 20°C until completion of the reaction. The mixture is poured into water and the phases separated. The organic phase is concentrated under reduced pressure and then purified by silica column chromatography (n-heptane/EtOAc) to obtain pure **43.1a.**

### Step 9:

**43.1a** is dissolved in MeOH and warmed to 40 °C, before addition of a solution of formic acid and oxalic acid in water. The resulting reaction mixture is stirred at 40 °C until completion of the reaction. The mixture is diluted with brine and extracted with MeTHF. The organic phase is separated, dried over Na₂SO₄ and concentrated under reduced pressure. The crude residue is dissolved in THF and cooled to 0 °C. A mixture of TBAF and acetic acid is added at 0 °C and the reaction mixture stirred at 30 °C until completion of the reaction. The solution is poured into a mixture of EtOAc and saturated NaHCOs solution, and the organic phase separated. The organic phase is washed with saturated NaHCOs and brine, dried over Na₂SO₄ and concentrated under reduced pressure. The residue is purified by silica column chromatography (n-heptane/EtOAc/MeOH) to obtain pure **46.1a.**

### Step 10:

**46.1a** is suspended in ACN and then methyl iodide is added. The resulting reaction mixture is stirred at 40°C until completion of the reaction. After filtration and concentration under reduced pressure crude **49.1a** is obtained.

### Step 11:

**49.1a** is added to a 1:1:1 mixture of toluene, 2-propanol and a 10% aqueous NaCl solution. The resulting reaction mixture is stirred at 35°C until completion of the reaction. After isolation, crude product is purified by recrystallization from 2-propanol, followed by multiple triturations with 2-propanol at RT for 1 h to obtain pure **52.1a**. Characterization by ICP-MS confirms **52.1a** as chloride salt.

### Example 3: Synthesis route of unsaturated DXPGₓ lipids

The synthesis route depicted below is for suited mono- and polyunsaturated DXPGₓ lipids. In the example below in particular the synthesis of DOPG₂ with oleyl lipid chains is shown. The synthesis proceeds via an intermediate of formula 4.2a. DOPG₂ is obtained in 13 synthesis steps in total and 4 steps from 4.2a and 34.1a.

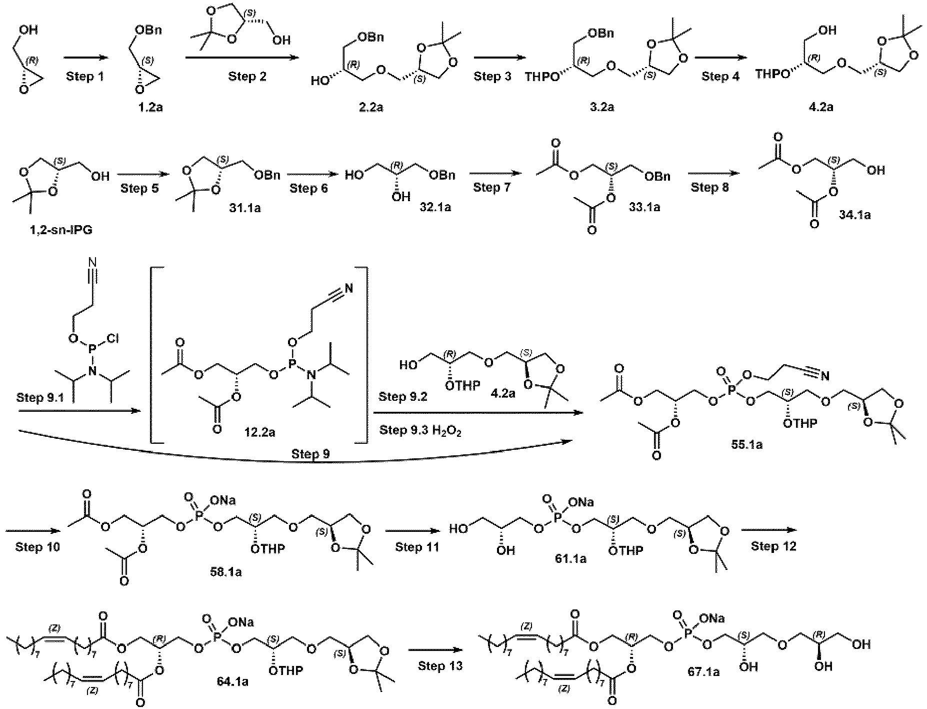

### Step 1:

(R)-(+)-glycidol is added to a suspension of NaH in DMF at 0 °C, followed by BnBr. The resulting reaction mixture is warmed to RT and stirred until completion of the reaction. Afterward, water is added, and the mixture extracted with DCM. The organic layer is washed with water and brine, dried over Na₂SO₄, filtered and concentrated under reduced pressure. The resulting residue is purified by silica gel chromatography (n-Heptane/Ethyl acetate) to obtain **1.2a.**

### Step 2:

**1.2a** is dissolved in DMF, then sn-1,2-IPG is added, followed by tBuOK at 0-5 °C and the resulting reaction mixture is stirred at RT until completion of the reaction. Then, the reaction mixture is poured into ice water and extracted with EtOAc. The organic phase is washed with brine, dried over Na₂SO₄ and concentrated under reduced pressure to obtain crude **2.2a**.

### Step 3:

Dihydropyran is added to a solution of **2.2a** and PPTS in DCM and the resulting reaction mixture is stirred at RT until completion of the reaction. The mixture is diluted with diethylether and washed with brine, followed by drying over Na₂SO₄ and concentration under reduced pressure to obtain crude **3.2a.**

### Step 4:

**3.2a** is added to a suspension of Pd/C in EtOH/DCM, followed by degassing. The resulting mixture is stirred under H₂ (0.5 MPa) at RT until completion of the reaction. The mixture is filtered and the filtrate concentrated under reduced pressure. Crude **4.2a** is purified by silica gel chromatography (n-Heptane/Ethyl acetate).

### Step 5:

(S)-(+)-1,2-lsopropylideneglycerol is dissolved in DMF, then BnBr added and the solution cooled to 0 °C. tBuONa is carefully added and the resulting reaction mixture is stirred at 25 °C until completion of the reaction. The mixture is poured into ice water and the pH adjusted to 6-7 with aqueous HCI, followed by extraction with EtOAc. The organic phase is washed with brine, dried over Na₂SO₄ and concentrated under reduced pressure to obtain crude **31.1a**.

### Step 6:

**31.1a** is dissolved in MeOH and the solution cooled to 0 °C. Concentrated HCl is carefully added and the resulting reaction mixture is stirred at 20 °C until completion of the reaction. The mixture is quenched with saturated aqueous K₂CO₃ solution to adjust the pH to 7-8, followed by extraction with EtOAc. The organic is dried over Na₂SO₄ and concentrated under reduced pressure to obtain crude **32.1a**.

### Step 7:

Pyridine and DMAP are added to a solution of **32.1a** in DCM, which is then cooled to 5 °C. Acetyl chloride is carefully added, and the resulting reaction mixture is stirred at 20 °C until completion of the reaction. The mixture is poured into DCM and washed with water. The organic phase is concentrated under reduced pressure to obtain crude **33.1a**.

### Step 8:

Pd/C is suspended in EtOH and then **33.1a** and DCM are added and the reactor flushed with H₂. The resulting reaction mixture is stirred under H₂ (5 bar) at 25°C until completion of the reaction. The mixture is filtered and the filtrate is concentrated under reduced pressure to obtain crude **34.1a**, which is recrystallized several times from n-heptane.

### Step 9:

DIPEA is added to a suspension of **34.1a** in MeTHF and DCM, then then mixture is cooled to -15 °C. 2-Cyanethyl-N,N-diisopropylchlorphosphoramidite (PAMCI) is dissolved in DCM and the solution cooled to -20 °C, before addition to the reaction mixture. The resulting mixture is stirred for 1 h at -15 °C, followed by 1.5 h at 0°C and 1 h at 20 °C. Then 4,5-dicyanoimidazole (DCI) and **4.2a** are dissolved in MeTHF and the solution slowly added at 20 °C to the reaction mixture. If necessary, an additional amount of DCI is added as solution in DCM after stirring for 1 h, then the whole mixture is stirred overnight at 20 °C. Then aqueous 35% w/w H₂O₂ solution is added at 0 °C over 1 h and then stirred at 20 °C for another 1 h. The reaction mixture is diluted with 5 wt% aq. Na₂S₂O₅ solution and extracted with MeTHF. The organic phase is dried over Na₂SO₄ and the solvents are removed under reduced pressure. The crude intermediate **55.1a** is purified by silica chromatography (DCM/acetone).

### Step 10:

DBU is added to a solution of **55.1a** dissolved in MeTHF and the reaction mixture stirred at RT for 2 h. Then Pd/C is added, and the obtained reaction suspension is stirred under H₂ atmosphere (1.2 bar) for 1 h. The reaction mixture is filtered over celite and washed with 1M aq. citric acid solution and organic phase separated. The organic phase is washed with 5 wt%. aq. NaHCO₃ solution, dried over Na₂SO₄ and concentrated under reduced pressure to obtain **58.1a**.

### Step 11:

A mixture of **58.1a**, K₂CO₃ and 2:1 MeOH/H₂O was stirred at RT until completion of the reaction. MeOH is removed under reduced pressure, then the aqueous phase extracted with MeTHF. The organic phase is separated, dried over Na₂SO₄ and concentrated under reduced to obtain crude **61.1a**.

### Step 12:

Pyridine and DMAP are added to a solution of **61.1a** in DCM, which is then cooled to 5 °C. Oleyl chloride is carefully added, and the resulting reaction mixture is stirred at 20 °C until completion of the reaction. The mixture is poured into DCM and washed with water. The organic phase is concentrated under reduced pressure to obtain crude **64.1a**.

### Step 13:

**64.1a** is dissolved in MeOH and warmed to 40 °C, before addition of a solution of formic acid and oxalic acid in water. The resulting reaction mixture is stirred at 40 °C until completion of the reaction. The mixture is diluted with brine and extracted with MeTHF. The organic phase is separated, dried over Na₂SO₄ and concentrated under reduced pressure. The crude residue is purified by silica column chromatography (n-heptane/EtOAc/MeOH) to obtain pure **67.1a**.

### Example 4: Synthesis, characterization and stability of CLG₂

HPLC/CAD measurements revealed presence of only minor lipid-related impurities (8.1 Area%) in the produced batch. The batch was investigated in indicative storage stability studies using HPLC/CAD. CLG₂ is stable in its powder form for at least 1 year when stored at -20°C. In contrast to NLG₂, CLG₂ could also be stored in organic solvents (e.g., chloroform) at temperature ranges from -20 to 2-8°C for at least 9 months.

CLG₂ is readily soluble in chloroform as a clear solution with at least 10 mg/ml. Methanol and isopropanol should be avoided since both solvents affected chemical stability of NLG₂. However, decomposition of CLG₂ in ethanol was less pronounced.

### Example 5: Preparation of liposomes

Liposomes were prepared as described elsewhere in detail (Hossann et al. 2021, Petrini et al. 2021).

Chol, DPPC and DSPC were acquired from Corden Pharma Switzerland LLC (Liestal, Switzerland). HSPC, NBD-PE, and RhPE were purchased from Avanti Polar Lipids (Alabaster, AL, USA). ADU-S100 (ammonium salt) was bought from MedChemExpress (Monmouth Junction, NJ, USA). CF free acid were bought from Sigma Aldrich (Darmstadt, Germany). All other chemicals were either from Sigma Aldrich GmbH (München, Germany) or Carl Roth GmbH (Karlsruhe, Germany). Unless stated, all the buffers and solutions mentioned in the study were prepared with ultra-purified deionized water from an ultrapure water system MilliQ Advantage from Merck Millipore (Darmstadt, Germany).

Liposomes were prepared with the lipid film hydration and extrusion method. The lipid components with the desired ratios were dissolved in either pure CHCl₃ or CHCl₃/MeOH 9:1 (vol/vol) followed with solvent removal to obtain a homogeneous and dry lipid film. Lipid films were stored at room temperature under vacuum until being hydrated in the desired solution (e.g., HBS pH 7.4, 100 mM CF pH 7.2, 300 mM citric acid pH 4). Dispersions were manually extruded (Avestin, Ottawa, Canada) 17 times through 200, 100, or 50 nm polycarbonate filters (Cytiva, MA, USA; and Avestin, Ottawa, Canada) at 60°C. Nonencapsulated small molecules were removed by size exchange column chromatography using HBS pH 7.4 as eluent. DOX was encapsulated to preformed liposomes by active loading. For passive encapsulation of ADU-S100, the dry lipid film was hydrated with a physiological sucrose-containing buffer pH 7 with 5 mg/ml ADU-S100. Additionally, liposomes with ADU-S100 underwent freezing and thawing cycles after the extrusion.

### Example 6: Biophysical characterization of liposomes

Liposome batches were characterized as described elsewhere (Hossann et al. 2007, 2010, 2021). Vesicle size (hydrodynamic diameter as z-average), polydispersity index (PDI), and zeta potential were measured by dynamic light scattering (DLS) with a Zetasizer Nano ZS (Malvern Instruments, Worcestershire, UK). Samples were diluted 50-times in 0.2 µm filtrated 0.9% saline. The desired batch specifications were specified to be <200 nm and <0.20 for z average and PDI, respectively. The solid gel to liquid disorder phase transition temperature (Tₘ) was analyzed using a Mettler Toledo DSC 822e (Giessen, Germany). Liposomal dispersions were sealed in aluminum crucibles for this purpose. Temperature dependent drug release (TDR) profile and release kinetics measurements in HBS pH 7.4 or fetal calf serum (FCS, Sigma Aldrich, Darmstadt, Germany) were performed in a Cary Eclipse fluorescence spectrometer (Varian Inc., Palo Alto, CA, USA). Content of the encapsulated compound in a liposomal batch was either performed with HPLC or fluorescence spectroscopy.

### Example 7: Compatibility testing of NLG₂ and/or NLG₂ with selected lipid excipients

Compatibility testing of NLG₂ and CLG₂ with selected binary and ternary lipid mixtures used for classical Chol-containing liposomes and TSL formulations was performed. Liposome preparation and characetrisation was performed in accordance with examples 5 and 6.

### Example 7.1: Binary mixtures of NLG₂, CLG₂ and DPPG₂

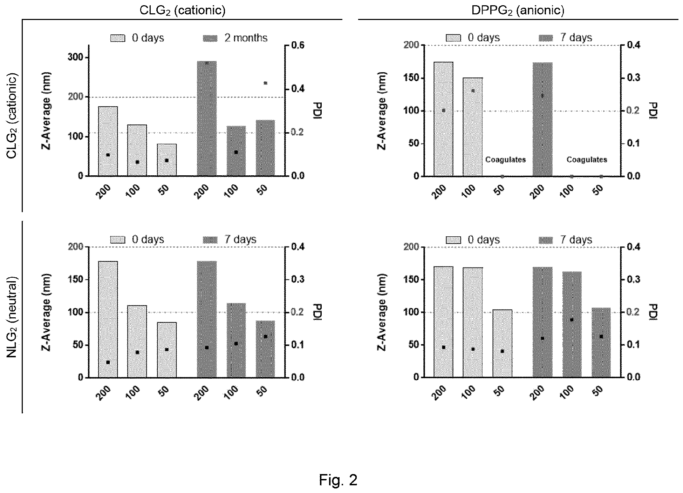

The feasibility of NLG₂, CLG₂ and DPPG₂ to form liposomes in binary mixtures was investigated at physiological pH (HBS pH 7.4). Extrusion was performed with either 50 nm, 100 nm, or 200 nm filter membranes to generate liposomes differing in vesicle size. Each 2-component system contained equal mol% of lipids. Additionally, feasibility of vesicle formation using pure NLG₂ or CLG₂ was tested. All resulting formulations were characterized by DLS after preparation and 7 days storage at 2-4°C. Results are depicted in Figure 2.

Overall, liposome formation was possible for CLG₂ and CLG₂/NLG₂ 1:1 (vol/vol). Vesicle size had no influence on the dispersion stability (z average/PDI) of CLG₂/NLG₂ 1:1 (vol/vol) batches. Oppositely charged CLG₂ and DPPG₂ form preparations with suboptimal characteristics (e.g., PDI > 0.2) and tend to form coagulates for smaller vesicle sizes. CLG₂ liposomes could be formed but -25% of the lipid was already hydrolyzed after preparation. After 2 months only the 100 nm extruded liposomes kept their integrity. Formation of NLG₂ liposomes was not successful at pH 7.4 but using NaOAc pH 4 and ultrasound resulted in liposomes (data not shown). Pure DPPG₂ was not tested since Lindner et al. previously showed that DPPG₂ only formed liposomes (DPPC/DSPC matrix) when incorporated from 0 to 70 mol% (Lindner et al. 2004).

### Example 7.2: Cholesterol-containing liposomes

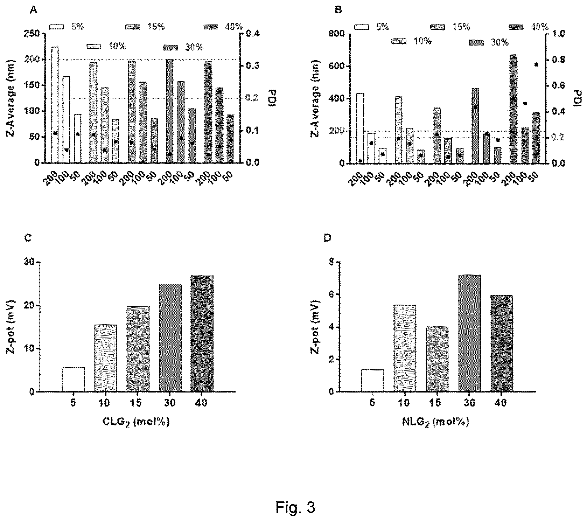

The compatibility of NLG₂ and CLG₂ with a phosphatidylcholine (PC)/cholesterol (Chol) matrix was investigated. PC/Chol is commonly used in classical, non-thermosensitive formulations (e.g., Caelyx^{®}). An overall Chol content ≥30 mol% is applied to suppress the phase transition of the PC by formation of liquid-ordered phase of the bilayer membrane. Distinct batches using a ternary mixture of PC/Chol/AXGₓ 60-x:40:x (mol/mol) (x = 5, 10, 15, 30, 40, PC = DPPC, HSPC, AXGₓ = NLG₂, CLG₂) were prepared using HBS pH 7.4. Results are depicted in Figure 3.

All CLG₂-containing formulations demonstrated optimal values for z average and PDI and dispersion stability was proven for at least 10 days at 2-8°C. Batches with >15 mol% of CLG₂ showed no signs of sedimentation. Batches that showed sedimentation could be homogenized again by mixing without affecting z average and PDI. The zeta potential increased from 5 mV to -25 mV with increasing content of CLG₂ from 5 to 40 mol%, respectively. Comparable results were obtained independent from usage of HSPC or DPPC. Binary mixtures of Chol/CLG₂ 1:1 (mol/mol) also resulted in liposomes within specification (z-average <200 nm, PDI <0.20) and maintained dispersion stability for at least 7 days.

In contrast, NLG₂ exhibited only partial compatibility to ternary PC/Chol/NLG₂ mixtures at physiological pH. Promising results were obtained for batches containing ≤15 mol% NLG₂ that had been extruded with 50 or 100 nm extrusion membranes. The effect of NLG₂ content on the zeta potential was less clear as observed before with CLG₂ but increased from ~1 mV to ~6 mV for batches with 5 and 40 mol% NLG₂, respectively. Comparable results were obtained whenever HSPC was replaced with DPPC. Batches within specification with a NLG₂ content of >15% were obtained when the lipid film was hydrated with an acidic buffer (data not shown), probably due to the stabilizing effect of the cationic surface charge. This fits to the results obtained with NLG₂-based TSL formulations in the pH titration assay. Binary mixtures of Chol/NLG₂ 1:1 (vol/vol) formed batches when the lipid film was hydrated with HBS pH 7.4.

### Example 7.3: Ternary mixtures (CLG₂/NLG₂/Chol)

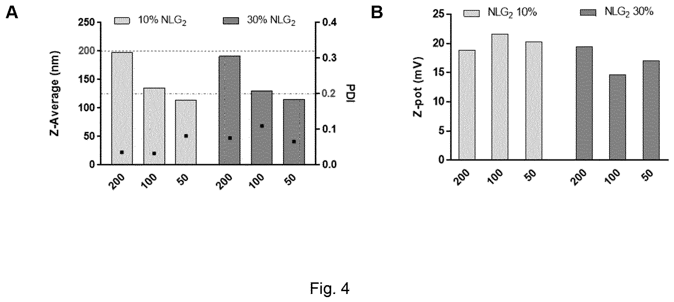

The compatibility to use both new excipients, NLG₂ and CLG₂, in Chol-containing liposomal formulations was investigated next. Mixing both in distinct ratios allows to adjust the positive zeta potential of the resulting liposomes while keeping the diglycerol content on the liposomal surface constant. Cholesterol does not contribute to the surface composition since it is incorporated in the lipophilic part of the membrane bilayer (Javanainen et al. 2017). Batches using a ternary mixture of either CLG₂/NLG₂/Chol 50:10:40 (mol/mol) or CLG₂/NLG₂/Chol 30:30:40 (mol/mol) were prepared using HBS pH 7.4. Extrusion was performed with membranes with a pore size of either 50, 100, or 200 nm, respectively. The results are depicted in Figure 4.

Both lipid compositions resulted in liposomes within specification independent from the used extrusion membrane. Presence of NLG₂ induces sedimentation of the liposomes, but batches could be stored at 2-8°C for 7 days and resulted in no change in vesicle size or PDI after vortexting (data not shown). The zeta potential decreases when the CLG₂ content decreased from 50 to 30 mol%.

### Example 7.4: Ternary mixtures DPPC/DSPC/AXGₓ

Finally, the compatibility of AXGₓ in the Chol-free DPPC/DSPC matrix used in TSL formulations was tested. Distinct batches of DPPC/DSPC/AXGₓ 80-x/20/x (mol/mol) x = 5, 10, 20, 30, 40, 50, 60, 70, 75 & AXGₓ = NLG₂, CLG₂ were prepared. DSPC content was fixed to 20 mol% to achieve a comparable fatty acid composition in the TSL membrane to be palmitic acid to stearic acid 80:20 (mol/mol), as done in lipid compatibility studies with the anionic DPPG₂ *in vitro* (Lindner et al. 2004) and *in vivo* (Hossann et al. 2021). To exclude any potential impact of additional molecules (i.e., loading of an API), lipids had been hydrated in HBS pH 7.4 to form the intra- and extra-liposomal medium. Liposomes with ≤30 mol% NLG₂ or ≤20 mol% CLG₂ could be successfully formulated, yielding batches within size specification (z-average < 200 nm, PDI < 0.20). These batches were dispersion stable for at least 7 days at 2-8°C without a notable change in vesicle size or PDI.

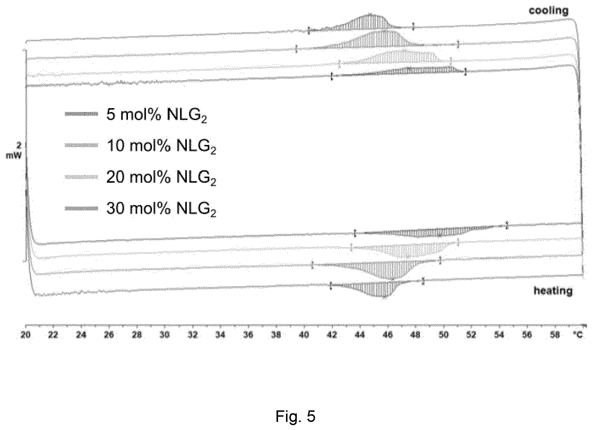

The effect of the NLG₂-content in DPPC/DSPC/NLG₂ 80-x/20/x (mol/mol) on the phase transition was investigated by DSC. The phase transition peak was broadened, and the peak maximum (Tₘ) was shifted towards higher temperatures with increasing NLG₂ content (Figure 5, Table 1), respectively. Tₘ decreased from 49.7°C to 42.1°C if cationizable NLG₂ is replaced by the anionic DPPG₂ in the 30 mol% formulation.

**Table 1: Phase transition temperature of NLG₂-based TSL formulations. Data for the DPPG₂-containing formulations from Petrini et al. 2021.**

| Lipid composition (mol/mol) | Phase transition peak maximum | |
|---|---|---|
| | Heating phase (°C) | Cooling phase (°C) |
| DPPC/DSPC/NLG₂ 75/20/5 (5 mol% NLG₂) | 45.72 | 44.74 |
| DPPC/DSPC/NLG₂ 70/20/10 (10 mol% NLG₂) | 46.31 | 45.76 |
| DPPC/DSPC/NLG₂ 60/20/20 (20 mol% NLG₂) | 47.44 | 47.24 |
| DPPC/DSPC/NLG₂ 50/20/30 (30 mol% NLG₂) | 49.68 | 47.56 |
| DPPC/DSPC/DPPG₂ 70/25/5 | 44.5 ± 0.2 | - |
| DPPC/DSPC/DPPG₂ 50/20/30 | 42.1 ± 0.4 | - |

### Example 8: Characterization of DPPC/DSPC/AXGₓ-based TSL formulations

### Example 8.1: pKₐ value of NLG₂-based TSL

A binding assay using the anionic fluorescent dye 2-(p-toluidino)-6-naphthalenesulfonic acid (TNS) was adapted from a described protocol (Carrasco et al. 2021). Objective was to determine the pKₐ value of saline-loaded DPPC/DSPC/NLG₂ 80-x/20/x (mol/mol) liposomal batches. TNS was purchased from Abcam (Cambridge, UK). Briefly, 50 µl of liposomes (75 µM total lipids) were mixed with 20 µl TNS stock solution (300 µM) and 1 ml buffer solutions with a pH ranging from 2 to 11, respectively. Osmolarities of the pH buffers were adjusted to approx. 287 mOsmol/kg. The fluorescence intensity was measured immediately with a Cary Eclipse fluorescence spectrometer (Varian Inc., Palo Alto, CA, USA) in 1 ml silica glass cuvettes. The data was fitted with GraphPad using the Henderson-Hasselbalch equation to obtain the pKₐ.

NLG₂-content had a negligible effect on pKₐ with 6.013, 6.095, and 5.807 for TSL with 10 mol%, 20 mol%, and 30 mol%, respectively. The measured pKₐ values are comparable to LNP formulations with the cationizable lipids DODAP (5.62) and DLin-MC3-DMA (6.57) (Carrasco et al. 2021).

### Example 8.2: Effect of pH on dispersion stability NLG₂-containing TSL

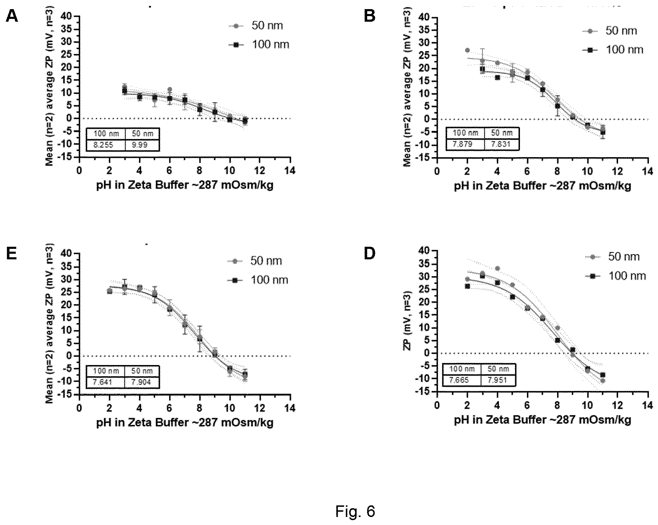

The effect of the extraliposomal pH value on the dispersion stability of DPPC/DSPC/NLG₂ 80-x/20/x (mol/mol) (x = 5, 10, 20, 30) batches was investigated by pH titration and subsequent measurement of z-average, PDI and zeta potential by DLS. Batches differing in vesicle size had been prepared by extrusion using membranes of either 50 or 100 nm pore size. All batches showed an effect of the pH value on the zeta potential of the liposomes (Figure 6). The change in zeta potential (*Δ*zeta potential = zeta potential_{pH3} - zeta potential_{pH11}) increased with increasing content of cationizable NLG₂ in the formulation. Vesicle size for individual lipid compositions had a negligble effect on the zeta potential results.

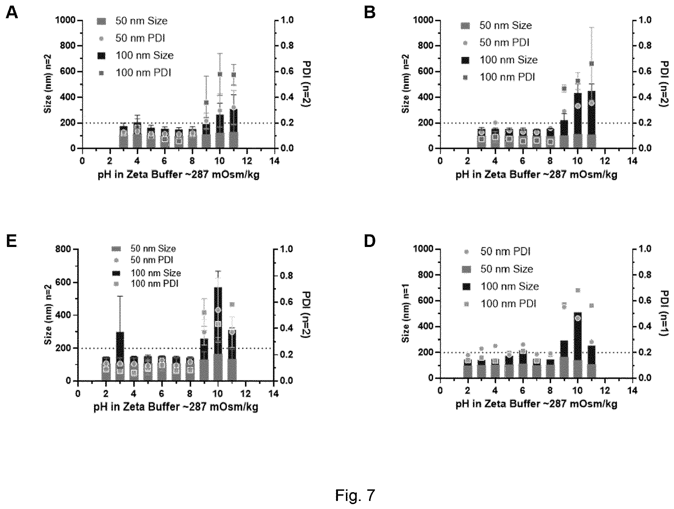

All tested formulations showed a stable z average and PDI in the pH range from 3 to 8 but showed a tendency for aggregation at pH ≥ 9 (Figure 7). All liposomal formulations lost their cationic surface charge around pH 9 where the zeta potential became neutral. Even 30 mol% NLG₂ in the neutral form was not sufficient to avoid aggregation. Anionic liposomes composed of DPPC/DSPC/DPPG₂ 50:20:30 (mol/mol) showed a similar behavior (data not shown). Liposomes lose their strong negative zeta potential and showed substantial increase in vesicle size and PDI when the extraliposomal pH was decreased to strongly acidic conditions (pH ~2). This demonstrates that liposomes containing a diglycerol-based surface coating instead of a PEGylation need a surface charge (either anionic or cationic) to maintain dispersion stability. Therefore, electrostatic repulsion due to the surface charge is the mechanism of diglycerols to maintain dispersion stability and not providing steric hinderance as proposed for PEGylation (Lasic et al. 1992). However, the absence of a steric barrier might be beneficial for the interaction between liposomes and the target cell.

### Example 8.3: In vitro complement activation of AXGₓ-based TSL

Complement activation, fast clearance due to serum protein adsorption and immunological recognition limits the clinical translation of charged DDS (Chonn et al. 1991, Dezsi et al. 2014, Knudsen et al. 2015, Szebeni et al. 2012).

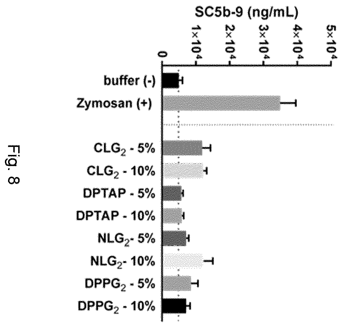

The *in vitro* complement activation of selected liposomal formulations was investigated with an enzyme-linked immunosorbent assay (ELISA) as described in Lokerse et al. 2021. Results are depicted in Figure 8. Liposomes containing the cationic CLG₂ (5 mol%, 10 mol%) and the cationizable NLG₂ (5 mol%, 10 mol%) showed complement activation. In contrast, cationic liposomes containing DPTAP (i.e., 5 mol%, 10 mol%) resulted in SC5b-9 values only slightly higher than the negative control. Liposomes containing the anionic DPPG₂ (5 mol%, 10 mol%) showed also complement activation comparable to the literature (Lokerse et al. 2021). Increasing the content of the anionic excipient DPPG₂ from 5 to 30 mol% in DPPC/DSPC/DPPG₂ TSL formulations notably reduced the *in vitro* complement activation, but it was still more pronounced than PEGylated TSL formulations. However, this had no negative effect on *in vivo* application of DPPG₂-based TSL to rodent and non-rodent species (Hossann et al. 2021).

### Example 8.4: Composition of the protein corona of AXGₓ-based TSL

Lokerse et al. 2021 demonstrated that the molar content of DPPG₂ influenced the composition of the protein corona of DPPC/DSPC/DPPG₂ 80-x/20/x (mol/mol) (x = 5, 10, 20, 30) liposomes. Here, the effect of different AXGₓ-based TSL formulations was investigated and compared to standard formulations.

### Example 8.5: Binding of NLG₂-based TSL to B16 cells (live cell fluorescence microscopy)

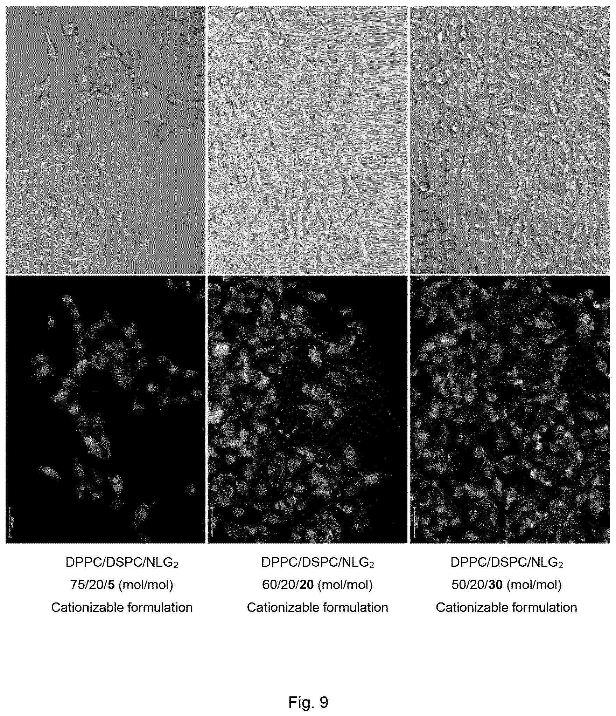

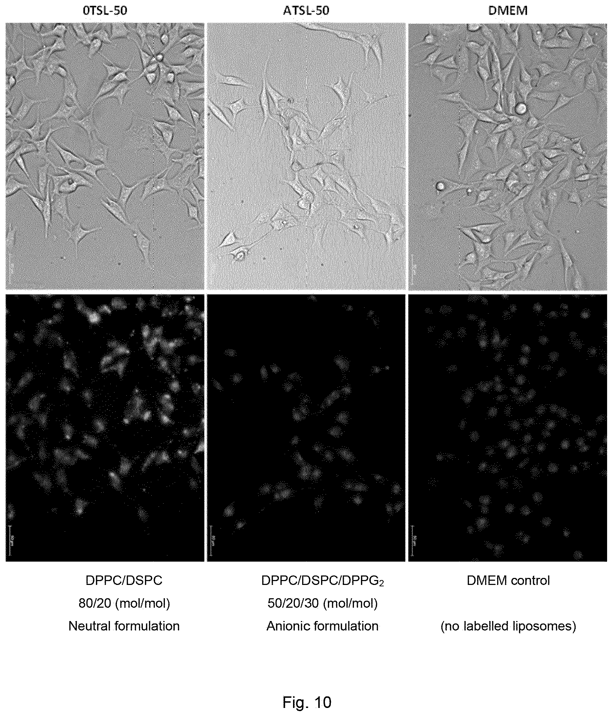

Several liposomal formulations were investigated *in vitro* for their binding to B16 cells. The respective lipid film contained 0.3 mol% of the fluorescent dye 1,2-dipalmitoyl-sn-glycero-3-phosphoethanolamine-N-(7-nitro-2-1,3 ben-zoxadiazol-4-yl) (NBD-PE, green) and was hydrated with HBS pH 7.4. Extrusion was performed with 50 nm filter membranes. Live cell fluorescence microscopy was performed as reported elsewhere (Petrini et al. 2021). Adherent mouse melanoma cells (B16 F10) were cultured in Dulbecco's Modified Eagle Medium (DMEM, ATCC, VA, USA) supplemented with 10% fetal bovine serum and 1% penicillin-streptomicin at humidified atmosphere containing 95% air and 5% CO₂ at 37 °C. Cell detachment was achieved by incubation with 0.25% trypsin at 37°C/2 min. Cell passages were performed when reached to 70-80% confluence (every 2-3 days). 25,000 cells were seeded in each well of a chambered coverslip for the live-cell fluorescence microscopy experiments. After 24 h, the culture media was removed and replaced by 250 µL of 1 mM liposome samples. Past 30 min, 50 µL of 0.5 µM Lysotracker Red in FBS-free DMEM were added. The medium was removed 30 min later and, after washing the cells with FBS-free DMEM, 300 µL of Hoechst 5 µg/mL in PBS were added. 5 min later, it was removed, and the cells were washed with PBS. A Leica wide-field microscope was used to observe the cells and the images were acquired with the Leica LAS X image software (Leica, Germany). Pictures were treated on imaged software (version 1.8.0_172) afterward. The results are depicted in Figure 9 (cationizable formulation) and Figure 10 (controls).

The cationizable DPPC/DSPC/NLG₂ liposomes localized mainly on the tumor cell surfaces. The intensity of the signal correlated with the NLG₂ content of the formulations confirming the assumption that cationizable TSL with a higher zeta potential would bind more intensely to the negatively charged surface of tumor cells. Lysotracker (red) stains acidic organelles in particular lysosomes. Orange appears upon red and green overlay, which can be interpreted as internalization of the labelled liposomes. This was seldom spotted, but relatively more noticeable for cells treated with DPPC/DSPC/NLG₂ 50:20:30 (mol/mol) liposomes. Co-localization with the Hoechst-stained nuclei (blue) was not observed.

Neutral DPPC/DSPC 80:20 (mol/mol) tended to aggregate due to their zeta -potential close to 0 mV. It is expected that larger particles sedimented quickly and mechanically deposited on top of the cells or on the surface of the slide. The latter could be responsible to the observed dotted pattern for this formulation.

Images of cells treated with the anionic DPPC/DSPC/DPPG₂ 50:20:30 (mol/mol) liposomes showed no green fluorescence, indicating that they were mostly washed away during the staining procedure. This is in good agreement with results from Petrini et al. (2021) who showed that cationic TSL (DPPC/DSPC/DPTAP/DPPG₂) target tumor and endothelial cells considerably more than anionic TSL (DPPC/DSPC/DPPG₂). As expected, experiments with cells incubated only with DMEM cell culture media showed no visible green NBD signal.

Overall, cationic or cationizable TSL showed superior binding to cell than neutral or anionic TSL.

### Example 8.6: Binding of AXGₓ-based TSL to THP-1 cells (FACS)

Distinct liposomal formulations differing in their lipid composition and zeta potential were prepared *for in vitro* cell binding studies using a fluorescent activated cell sorter (FACS). All formulations were labelled with 0.1 mol% 1,2-dipalmitoyl-sn-glycero-3-phosphoethanolamine-N-(lissamine rhodamine B sulfonyl) (RhPE) as fluorescent dye. The human monocytic cell line THP-1, derived from an acute monocytic leukemia patient were seeded in a 96-well plate in an appropriate medium containing 10% FCS. 0.6 mM of a Rh-PE labelled liposomal sample was added to each well. FACS analysis was performed at specified time points.

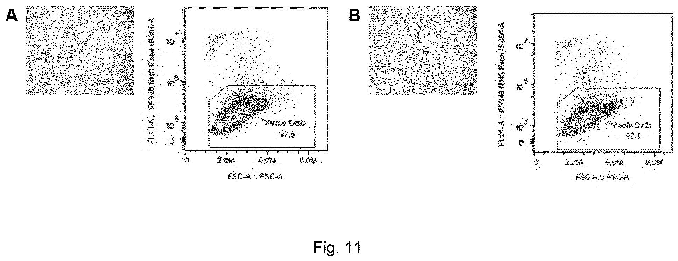

Reducing the content of anionic DPPG₂ from 30 mol% to 0 mol% increased the cell binding of the TSL formulation (Table 2, #1-4), probably due to a reduction of the electrostatic repulsion of the liposome and the cell. A higher content of cationic lipid (i.e., CLG₂, DPTAP) induced higher cell binding (Table 2, #5,6,9,10). Liposomes with CLG₂ showed better binding than liposomes with DPTAP (Table 2, e.g., #5 & #9). Liposomes with a strong permanent cationic charge (CLG₂) outperformed liposomes with a cationizable charge (NLG₂) in binding of cells (Table 2, #6 & #8). Interestingly, visual inspection of incubated cells showed cell agglutination in vials with the cationic formulation DPPC/DSPC/CLG₂ 70:20:10 (mol/mol) (Figure 11), while all other formulations looked like the control (no agglutination).

**Table 2: THP-1 cell binding of DPPG₂-, AXGₓ-, and DPTAP-based TSL formulations. All liposomes had been labelled with 0.1 mol% RhPE as fluorescent dye to allow quantification of cell-liposome binding by FACS. MFI: median fluorescent intensity.**

| # | Lipid composition | Type of DDS | z average (nm) | zeta potential (mV) | MFI (a.u.) |
|---|---|---|---|---|---|
| 1 | DPPC/DSPC/DPPG₂ 50:20:30 | Anionic | 80.1 | -20.8 | 10.4 |
| 2 | DPPC/DSPC/DPPG₂ 70:20:10 | Anionic | 74.9 | -9.6 | 18.2 |
| 3 | DPPC/DSPC/DPPG₂ 75:20:5 | Anionic | 76.7 | -7.2 | 32.2 |
| 4 | DPPC/DSPC 80:20 | Neutral | 72.1 | -3.1 | 62.8 |
| 5 | DPPC/DSPC/CLG₂ 75:20:5 | Cationic | 76.7 | +6.0 | 117.0 |
| 6 | DPPC/DSPC/CLG₂ 70:20:10 | Cationic | 74.5 | +13.7 | 362.0 |
| 7 | DPPC/DSPC/NLG₂ 75:20:5 | Cationizable | 141.4 | +1.8 | 73.2 |
| 8 | DPPC/DSPC/NLG₂ 70:20:10 | Cationizable | 142.7 | +3.3 | 54.9 |
| 9 | DPPC/DSPC/DPTAP 75:20:5 | Cationic | 130.7 | +11.3 | 53.0 |
| 10 | DPPC/DSPC/DPTAP 70:20:10 | Cationic | 128,5 | +10.8 | 301.4 |

### Example 9: AXGₓ-based TSL loaded with small molecules

The simple formulation of small molecules into AXGₓ-based TSL was investigated next. The fluorescent dye carboxyfluorescein (CF), the cytotoxic drug DOX, and the stimulator of IFN genes (STING) agonist ADU-S100 were used as representative small molecules, respectively.

The carboxylic acid CF is the smallest of the three used compounds. CF is commonly applied in formulation development of liposomes to evaluate release rates. Fluorescence intensity is quenched at high concentrations. It can be loaded by passive encapsulation and was extensively investigated in the anionic DPPG₂-based TSL (Lindner et al. 2004, Hossann et al. 2007, 2010, 2021). At physiological pH, the molecule is negatively charged while being in its salt form.

DOX belongs to the class of anthracyclines and is a weak base with fluorescent properties. Fluorescence intensity is quenched at high concentrations. The drug is water soluble at acidic pH while being positively charged, but lipophilic in its unprotonated form. The molecule can be actively loaded into preformed liposomal formulations by pH, EDTA and ammonia gradients, respectively.

ADU-S100 is the largest of the three model compounds used and is a synthetic cyclic dinucleotide that activates the STING pathway. It is a negatively charged molecule when in salt form.

The small molecules had been loaded into liposomes and the resulting batches had been characterized regarding their vesicle size, PDI and compound release properties immediately after preparation.

### Example 9.1: NLG₂-based TSL passively loaded with CF

Several lipid films with the composition DPPC/DSPC/NLG₂ 80-x:20/x (mol/mol) with x = 5, 10, 20, 30, 40, 50, 60, 70, 80 had been incubated with an aqueous solution of 100 mM CF pH 7.2. Hydration of the film was possible with lipid compositions <50 mol% NLG₂. Hydrated films were extruded with membrane filters of either 50, 100, or 200 nm pore size, respectively, and subsequently purified with size exclusion chromatography (SEC). Only distinct formulations with a NLG₂ content ≤30 mol% met the quality criteria for z average and PDI. Increasing the NLG₂ content from 5 to 30 mol% led to an increase in the zeta potential of the formulations from 3 to 13 mV, respectively.

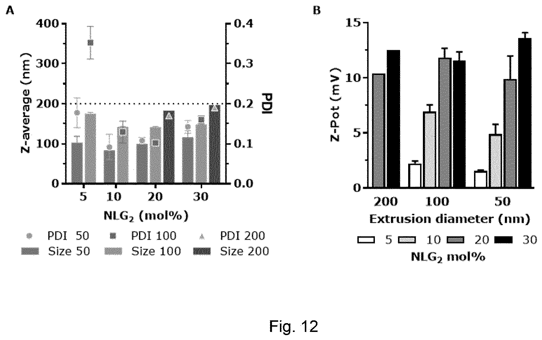

Vesicle size seems to affect the colloidal stability of the formulations (Figure 12). For example, using 200 nm filter membranes during extrusion did not yield monodisperse size distribution for lipid compositions of DPPC/DSPC/NLG₂ 75:20:5 and 70:20:10 (mol/mol), respectively, but formation of monodisperse liposomes was possible when using 50 nm and 100 nm filters. It seems that the membrane curvature has an impact on dispersion stability of the NLG₂-based TSL. Since decreasing vesicle size leads to an increased membrane curvature with more packing defects and loser packing of phospholipids (Hossann et al. 2010), this seems to be important for compatibility of NLG₂ to the membrane bilayer during passive loading with the anionic CF.

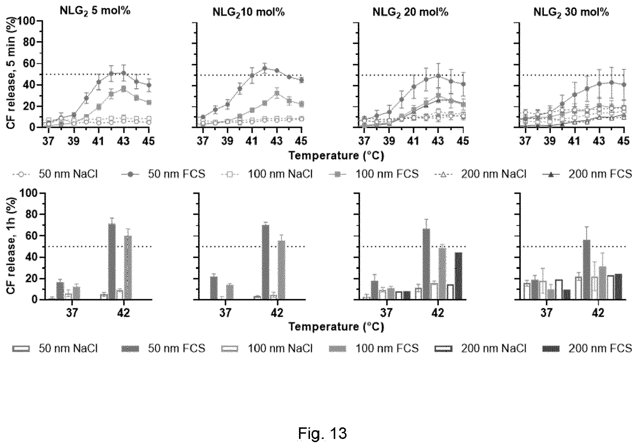

The temperature-dependent release (TDR) profiles of selected batches were measured either in FCS or in 0.9% saline (Figure 13), respectively. Presence of serum components was necessary to induce notable CF release from NLG₂-based TSL at T≥38°C during 5 min of incubation. Necessity of serum components to support CF release was previously shown also for TSL containing the anionic excipient DPPG₂ (Hossann et al. 2012). The TDR profile obtained for NLG₂-based TSL was increasingly less defined for higher NLG₂ content. As result, with increasing NLG₂ content the temperature where the maximum release occurred was slightly shifted towards higher temperatures accompanied by a slight reduction in amount of released CF during the incubation time of 5 minutes. The observation can be explained by the broadening of the phase transition with increasing NLG₂ content in a formulation (see example 7.4). Maximum CF release during 5 min at 42°C was -50% and -25% for NLG₂-based TSL batches extruded with 50 nm and 100 nm membranes, respectively. This is in clear contrast to the anionic DPPG₂-based TSL that showed an increase in CF release when the vesicle size was decreased (Hossann et al. 2010).

Time-dependent release assay results confirmed the TDR results and further demonstrated that the steepest increase in CF release was achieved within the first seconds of the experiment (data not shown). A behavior that was also demonstrated for TSL containing the anionic excipient DPPG₂ (Hossann et al. 2007). An immediate heat-triggered drug release is important for TSL formulations to be able to exploit the concept of intravascular drug release since the passage time through the heated tumor tissue is limited.

### Example 9.2: CLG₂-based TSL passively loaded with CF

Several lipid films with the composition DPPC/DSPC/CLG₂ 80-x:20/x (mol/mol) with x = 5, 10, 20, 30 had been incubated with an aqueous solution of 100 mM CF pH 7.2. CLG₂ contents >30 mol% were not tested because of the observed broadening of the phase transition with increasing NLG₂ content in a formulation (see example 7.4). Hydrated films were extruded with membrane filters of either 100, or 200 nm pore size, respectively, and subsequently purified with SEC.

Acceptable values for size (z average) of < 200nm and PDI (<0.20) were observed for two batches with 5 mol% CLG₂ (DPPC/DSPC/CLG₂ 75:20:5 (mol/mol)) and 10 mol% CLG₂ (DPPC/DSPC/CLG₂ 70:20:10 (mol/mol)) and usage of 100 nm pore-size filters during extrusion. The zeta potential showed a CLG₂-content dependent increase in charge from ~4 mV and ~12 mV for TSL with 5 mol% and 10 mol%, respectively.

An electrostatic interaction of the negatively charged CF with the positively charged CLG₂ during hydration might be responsible for the failure in formation of liposomes with acceptable quality when >10 mol% CLG₂ was applied. A strong interaction of drug with the lipids might interfere with the self-assembly process of the lipids when dispensed in the hydration solution.

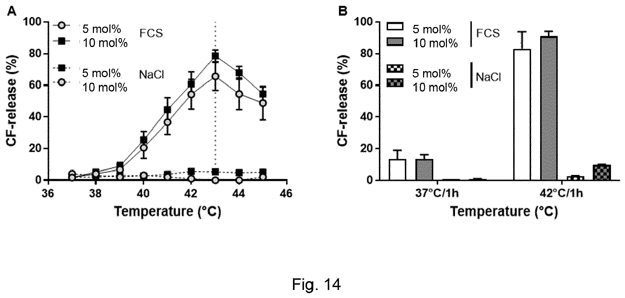

Two batches within specification were tested for their TDR profile in either FCS or 0.9% saline (Figure 14), respectively. As shown before for NLG₂-based TSL, presence of serum components was also important for CLG₂- based TSL to facilitate heat-triggered CF release. Importantly for *in vivo* application, both formulations showed a sufficient stability at 37°C/60 min in FCS and -80% CF release at 42°C/60 min, respectively. Additionally, negligible CF release in physiological saline under all tested conditions suggests a promising stability of both formulations during storage. Finally, CF release kinetics of both batches was performed in FCS with real time fluorescence spectroscopy and confirmed the TDR results (data not shown).

### Example 9.3: CLG₂-based TSL actively loaded with DOX

Lipid compositions with 5 mol% CLG₂ (DPPC/DSPC/CLG₂ 75:20:5 (mol/mol)) and 10 mol% CLG₂ (DPPC/DSPC/CLG₂ 70:20:10 (mol/mol)) were selected for testing active DOX loading, based on the positive results obtained in example 9.2. Hydration of the lipid film was done with 300 mM ammonium sulfate pH 5.5 and filter membranes with 100 nm pore size were used for the subsequent extrusion. Ammonium gradient was than formed by SEC with HBS pH 7.8. DOX loading was completed after 90 min using an incubation temperature of 37°C.

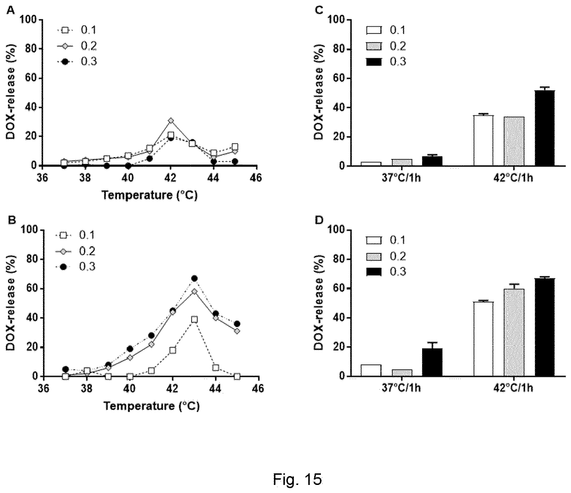

Distinct molar DOX:lipid ratios (0.1, 0.2, and 0.3) were tested for active DOX loading and the TDR profile was measured. Both CLG₂-based TSLs showed feasibility for active DOX loading and heat-triggered drug release (Figure 15). The amount of DOX released at elevated temperatures depended on the CLG₂ content of the formulation with 10 mol% CLG₂-based TSL (~40-70%/42°C/5min) being superior to the 5 mol% CLG₂-based TSL (-20-30%/42°C/5min). Moreover, the molar DOX:lipid ratio might also affect the amount of DOX released. However, this also impacted the TSL stability since both formulations loaded with a molar DOX:lipid ratio of 0.30 showed the largest DOX leakage at 37°C/60 min compared to the respective formulations with lower molar DOX:lipid ratios. An effect of drug:lipid ratio on TSL stability fits to published observations with DPPG₂-based TSL actively loaded with DOX or irinotecan (WO2022/008471), respectively.

The obtained data shows that active DOX loading and subsequent heat-triggered DOX release from CLG₂-based TSL is possible.

### Example 9.4: CLG₂-based TSL passively loaded with ADU-S100

Next, the feasibility of cationic CLG₂-based TSL for stable encapsulation and heat-triggered release of the synthetic cyclic dinucleotide ADU-S100 was tested. Because of the promising results obtained with CF and DOX, lipid compositions with 5 mol% CLG₂ (DPPC/DSPC/CLG₂ 75:20:5 (mol/mol)) and 10 mol% CLG₂ (DPPC/DSPC/CLG₂ 70:20:10 (mol/mol)) were selected for passive encapsulation. Using 5 mg/ml ADU-S100 for hydration of the lipid film yielded acceptable TSL batches. Furthermore, presence of 5% (wt/vol) saccharides (e.g., glucose, sucrose, and trehalose) were tested to act as potential stabilizers and to facilitate steric hindrance between the oppositely charged CLG₂ and ADU-S100.

DPPC/DSPC/CLG₂ 75:20:5 (mol/mol) batches hydrated with sucrose-containing buffer containing ADU-S100 with a molar drug:lipid ratio of 0.14 and extruded with 100 nm pore size filters showed promising results. Vesicle size (z average) and PDI was 83 ± 9 nm and 0.07 ± 0.03, respectively. The ADU-S100 encapsulation efficacy was 8.3 ± 3.1 % and the molar drug:lipid ratio was 0.012 ± 0.004. The formulation showed a promising storage stability and was store-able up to 2 months at 2-8°C.

ADU-S100 was released from the CLG₂-based TSL in a temperature-dependent fashion. The formulation released 62.7 ± 14.0% during 5 min at 42 °C. In contrast, only traces of ADU-S100 were released at 37°C during 1 hour in FCS.

Herein also the following items are disclosed.
Item 1. Oligoglycerol-containing lipid of Formula (I) 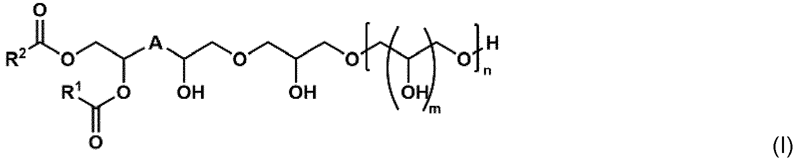
   wherein R¹ and R² each independently are a hydrocarbon group having 12 to 24 C-atoms;
   A = NR³ or N⁺R⁴R⁵;
   R³, R⁴ and R⁵ each independently are hydrogen or a hydrocarbon group having 1 to 5 C-atoms, and
   n = a number from 0 to 20, and
   m at each occurrence independently is 0 or 1.
Item 2. Oligoglycerol-containing lipid of Formula (I) 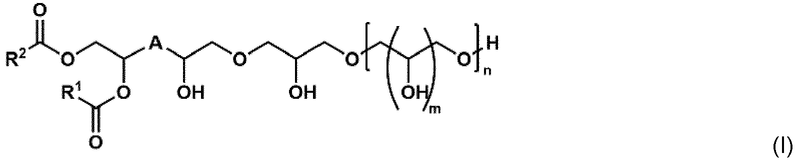
   wherein R¹ and R² each independently are a hydrocarbon group having 12 to 24 C-atoms;
   A = NR³ ;
   R³ is hydrogen or a hydrocarbon group having 1 to 5 C-atoms;
   n = a number from 0 to 20; and
   m at each occurrence independently is 0 or 1.
   Those compounds are also termed NLGₓ herein.
Item 3. Oligoglycerol-containing lipid of Formula (I) 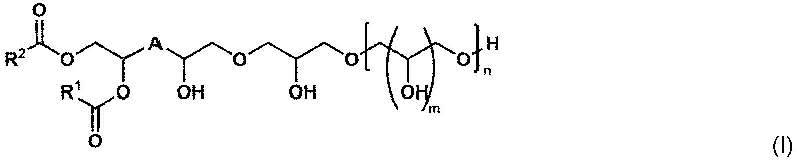
   wherein R¹ and R² each independently are a hydrocarbon group having 12 to 24 C-atoms;
   A = N⁺R⁴R⁵;
   R⁴ and R⁵ each independently are hydrogen or a hydrocarbon group having 1 to 5 C-atoms;
   n = a number from 0 to 20; and
   m at each occurrence independently is 0 or 1.
   Those compounds are also termed CLGₓ herein.
Item 4. Oligoglycerol-containing lipid according to any one of items 1-3,
   wherein n is a number from 0 to 10, preferably wherein n is a number from 0 to 5, more preferably wherein n is 0, 1 or 2, even more preferably wherein n is 0 or 1.
Item 5. Oligoglycerol-containing lipid according to any one of items 1 - 4,
   wherein n = 0.
Item 6. Oligoglycerol-containing lipid according to any one of items 1 - 5,
   wherein at each occurrence m = 1.
Item 7. Oligoglycerol-containing lipid according to any one of items 1 - 6,
   wherein A = NR³ and R³ = C1-C5-alkyl, in particular R³ = CH₃, or
   wherein A = N⁺R⁴R⁵ and R⁴ and R⁵ each independently are a C1-C5-alkyl, and in particular R⁴ = R⁵ =CH₃.
Item 8. Oligoglycerol-containing lipid according to any one of items 1 - 7,
   wherein R¹ and R² independently are a hydrocarbon group having 13 to 19 C-atoms and in particular, wherein R¹ and R² are a linear or branched C13- to C19-alkyl group.
Item 9. Oligoglycerol-containing lipid according to any one of items 1 - 8,
   wherein R¹ and R² independently are a mono- or polyunsaturated C13-C19₉ hydrocarbon group.
Item 10. Oligoglycerol-containing lipid according to any one of items 1 - 9,
   wherein R¹ and R² independently are a saturated C13-C19-alkyl group.
Item 11. Oligoglycerol-containing lipid according to any one of items 1-10,
   wherein R¹ and R² are a linear saturated C15 or C17 alkyl group.
Item 12. Oligoglycerol-containing lipid according to any one of items 1, 2 or 4 -11 having Formula (III) 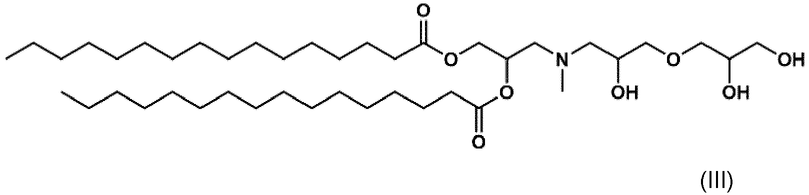 This compound is also termed NLG₂ herein.
Item 13. Oligoglycerol-containing lipid according to any one of items 1 or 3 - 11 having Formula (IV) 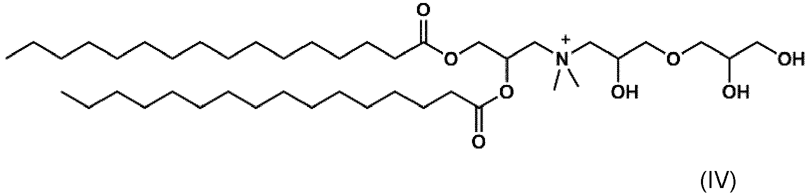 This compound is also termed CLG₂ herein.
Item 14. Oligoglycerol-containing lipid according to any one of items 1 - 13 having Formula (II) 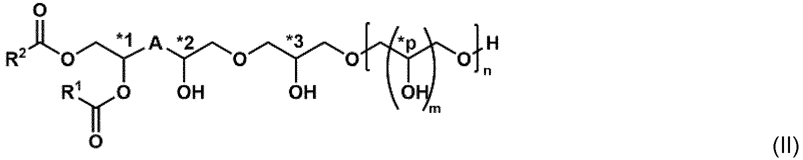 wherein the three stereogenic centers *1, *2 and *3 have a predetermined configuration and wherein *p denotes further stereogenic centers.
Item 15. Oligoglycerol-containing lipid according to item 14 having Formula (II),
   wherein stereogenic center *1 is in R-configuration, stereogenic center *2 is in S-configuration and stereogenic center *3 is in R-configuration.
Item 16. Oligoglycerol-containing lipid according to item 14 or 15 having Formula (II),
   wherein the stereogenic centers *p have a predetermined configuration.
Item 17. Oligoglycerol-containing lipid according to any one of items 14 - 16 having Formula (II), wherein the oligoglycerol-containing lipid is a single stereoisomer.
Item 18. A method for preparing an oligoglycerol-containing lipid according to any one of items 1 - 17, wherein the synthesis route includes an intermediate of Formula (V) or of Formula (V') 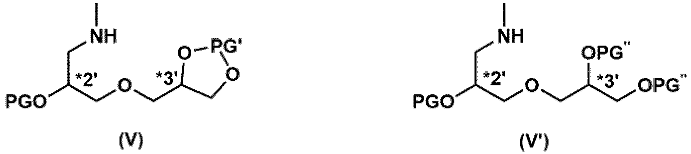 wherein PG, PG' and PG" are independently a protecting group.
Item 19. The method for preparing an oligoglycerol-containing lipid according to item 18, wherein PG in particular is benzyl (Bn), tetrahydropyranyl (THP), ethoxyethylacetal (EE), 2-methoxypropan-2-ylacetal (MOP), silyl or para-methoxybenzyl (PMB), preferably PG is benzyl (Bn) for the synthesis of an oligoglycerol-containing lipid of Formula (I), wherein R¹ and R² independently are a saturated hydrocarbon group; preferably PG is THP or silyl for the synthesis of an oligoglycerol-containing lipid of Formula (I), wherein R¹ and R² independently are a mono- or polysaturated hydrocarbon group; and/or wherein PG' is CH₂ (methylene acetal), CHCH₃ (ethylidene acetal), CHPh (benzylidene acetal), CHPhp-Ome (para-methoxybenzylidene acetal), C(CH₃)₂ (isopropylidene ketal) or Si*t*Bu₂ (di-tert-butylsilylene), Si(iPr)₂-O-Si(iPr)₂ (tetraisopropyldisiloxanylidene), preferably PG' is C(CH₃)₂ and/or wherein PG" in particular is benzyl (Bn), tetrahydropyranyl (THP), ethoxyethylacetal (EE), 2-methoxypropan-2-ylacetal (MOP), silyl or para-methoxybenzyl (PMB), preferably PG is benzyl (Bn).
Item 20. The method for preparing an oligoglycerol-containing lipid according to item 18 or 19, comprising reacting a compound of Formula (V) or of Formula (V') with glycidol.
Item 21. A liposome comprising at least one oligoglycerol-containing lipid according to any one of items 1-17.
Item 22. The liposome according to item 21 comprising at least 1 mol.- %, preferably at least 5 mol.-%, more preferably at least 10 mol.-% of oligoglycerol-containing lipids according to any one of items 1-17, based on the lipid content of the liposome.
Item 23. The liposome according to item 21 or 22 comprising at least 10 mol.- %, preferably at least 20 mol.-%, more preferably at least 50 mol.-% of oligoglycerol-containing lipids according to any one of items 1-17, based on the lipid content of the liposome.
Item 24. The liposome according to any one of items 21 - 23 comprising up to 100 mol.-%, preferably up to 80 mol.-%, more preferably up to 50 mol.-% of oligoglycerol-containing lipids according to any one of items 1-17, based on the lipid content of the liposome.
Item 25. The liposome according to any one of items 21 - 24, comprising cholesterol.
Item 26. The liposome according to any one of items 21 - 25, comprising cholesterol in an amount of from 1 mol.-%, preferably of from 30 mol.-% up to 70 mol.-%, preferably up to 60 mol.-%, based on the lipid content of the liposome.
Item 27. The liposome according to any one of items 21 - 26, further comprising at least one of phosphatidylcholine of formula (VII) 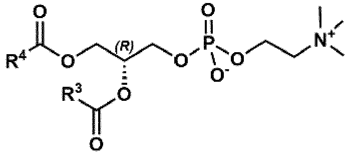 wherein R³ and R⁴ each independently represent a hydrocarbon group having from 12 to 24 carbon atoms.
Item 28. The liposome according to any one of items 21 - 27, comprising at least one of phosphatidylcholine of formula (VII) 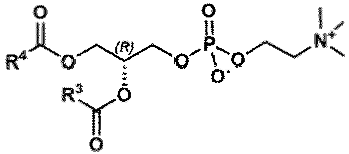 wherein R³ and R⁴ are each independently a saturated or mono- or polyunsaturated, preferably linear alkyl group, in particular a saturated linear alkyl group.
Item 29. The liposome according to any one of items 21 - 28, comprising at least one of 1,2-dipalmitoyl-*sn*-glycero-3-phospho-choline (DPPC) and 1,2-distearoyl-*sn-*glycero-3-phosphocholine (DSPC).
Item 30. The liposome according to any one of items 21 - 28, comprising
   (i) at least one oligoglycerol-containing lipid of Formula (I) according to item 2; and
   (ii) at least one oligoglycerol-containing lipid of Formula (I) according to item 3.
Item 31. The liposome according to 30, comprising
   (i) at least one oligoglycerol-containing lipid of Formula (I) according to item 2;
   (ii) at least one oligoglycerol-containing lipid of Formula (I) according to item 3; and
   (iii) at least one phosphatidylcholine of Formula (VII), in particular 1,2-dipalmitoyl-*sn*-glycero-3-phospho-choline (DPPC) and/or 1,2-distearoyl-*sn-*glycero-3-phosphocholine (DSPC).
Item 32. The liposome according to item 31, further comprising (iv) cholesterol.
Item 33. The liposome according to any one of items 21 - 32, comprising at least one phosphatidyloligoglycerol of Formula (VI) 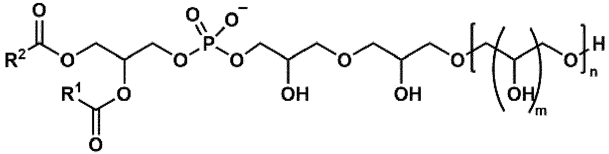 wherein R¹ and R² each independently are a hydrocarbon group having 12 to 24 C-atoms and wherein n is a number from 0 to 20, and m is 0 or 1.
Item 34. The liposome according to any one of items 21 - 33, comprising at least one phosphatidyloligoglycerol of Formula (VI') 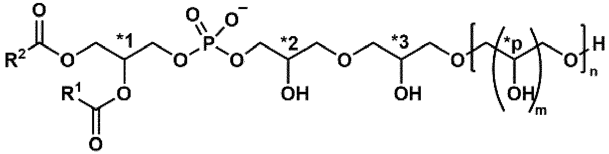
   wherein the three stereogenic centers *1, *2 and *3 have a predetermined configuration; and *p denotes further stereogenic centers; and
   wherein R¹ and R² each independently are a hydrocarbon group having 12 to 24 C-atoms; n is a number from 0 to 20; and
   m at each occurrence independently is 0 or 1 and in particular
   wherein stereogenic center *1 is in R-configuration, stereogenic center *2 is in S-configuration and stereogenic center *3 is in R-configuration and *p denotes further stereogenic centers.
Item 35. The liposome according to any one of items 21 - 34, comprising at least one phosphatidyloligoglycerol of Formula (VI") 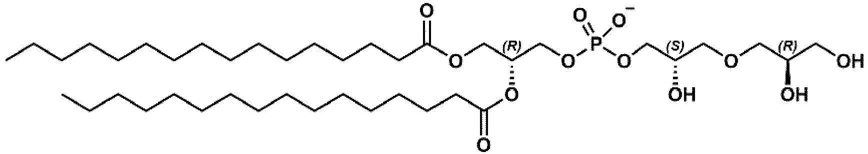
Item 36. The liposome according to any one of items 21 - 35, characterized in that it is a thermosensitive liposome.
Item 37. The liposome according to any one of items 21 -26, further comprising an active agent, in particular doxorubicin.

## Claims

1. Oligoglycerol-containing lipid of Formula (I) 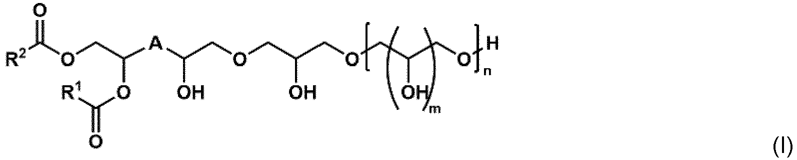
wherein R¹ and R² each independently are a hydrocarbon group having 12 to 24 C-atoms;
A = NR³ or N⁺R⁴R⁵;
R³, R⁴ and R⁵ each independently are a hydrocarbon group having 1 to 5 C-atoms, and
n = a number from 0 to 20, and
m is 0 or 1.

2. Oligoglycerol-containing lipid according to claim 1, wherein n = 0, 1 or 2 and in particular, wherein n = 0 or 1.

3. Oligoglycerol-containing lipid according to any one of claim 1 or 2, wherein at each occurrence m = 1.

4. Oligoglycerol-containing lipid according to any one of claims 1-3, wherein A = NR³ and R³ = C1-C5-alkyl, in particular R³ = CH₃; and/or
wherein A = N⁺R⁴R⁵ and R⁴ and R⁵ each independently are a C1-C5-alkyl, and in particular R⁴ = R⁵ = CH₃.

5. Oligoglycerol-containing lipid according to any one of claims 1-4, wherein R¹ and R² independently are a hydrocarbon group having 13 to 19 C-atoms and in particular, wherein R¹ and R² are a linear or branched C13- to C19-alkyl group.

6. Oligoglycerol-containing lipid according to any one of claims 1-5, wherein R¹ and R² independently are a mono- or polyunsaturated C13-C19 hydrocarbon group.

7. Oligoglycerol-containing lipid according to any one of claims 1-6, wherein R¹ and R² independently are a saturated C13-C19-alkyl group.

8. Oligoglycerol-containing lipid according to any one of claims 1-7 having Formula (II) 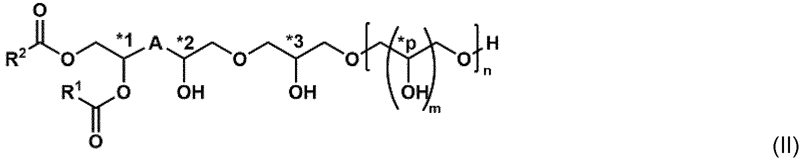 wherein the three stereogenic centers *1, *2 and *3 have a predetermined configuration and in particular wherein stereogenic center *1 is in R-configuration, stereogenic center *2 is in S-configuration and stereogenic center *3 is in R-configuration.

9. A method for preparing an oligoglycerol-containing lipid according to any one of claims 1-8, wherein the synthesis route includes an intermediate of Formula (V) or of Formula (V') 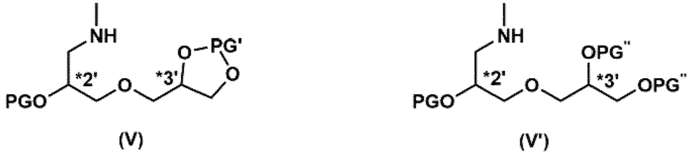 wherein PG, PG' and PG" are independently a protecting group.

10. A liposome comprising at least one oligoglycerol-containing lipid of Formula (I) according to any one of claims 1-8.

11. The liposome according to claim 10 comprising at least 1 mol.- of oligoglycerol-containing lipids according to any one of claims 1-8, based on the lipid content of the liposome.

12. The liposome according to any one of claims 10 or 11, further comprising cholesterol.

13. The liposome according to any one of claims 10 - 12, further comprising at least one of phosphatidylcholine of formula (VII) 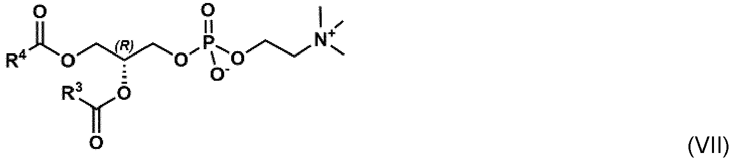
wherein R³ and R⁴ each independently represent a hydrocarbon group having from 12 to 24 carbon atoms; and preferably at least one of
1,2-dipalmitoyl-*sn*-glycero-3-phospho-choline (DPPC) and 1,2-distearoyl-*sn-*glycero-3-phosphocholine (DSPC).

14. The liposome according to any one of claims 10 - 13, comprising
(i) at least one oligoglycerol-containing lipid of Formula (I) wherein A = NR³ ; and
(ii) at least one oligoglycerol-containing lipid of Formula (I) wherein A = N⁺R⁴R⁵.

15. The liposome according to any one of claims 10 - 14, comprising at least one phosphatidyloligoglycerol of Formula (VI') 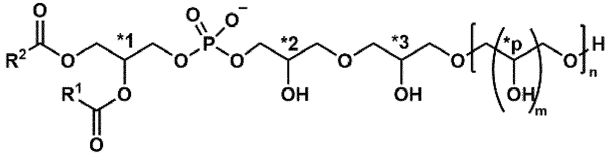
wherein the three stereogenic centers *1, *2 and *3 have a predetermined configuration; and *p denotes further stereogenic centers; and
wherein R¹ and R² each independently are a hydrocarbon group having 12 to 24 C-atoms; n is a number from 0 to 20; and
m at each occurrence independently is 0 or 1 and in particular
wherein stereogenic center *1 is in R-configuration, stereogenic center *2 is in S-configuration and stereogenic center *3 is in R-configuration and *p denotes further stereogenic centers.

16. The liposome according to any one of claims 10 - 15, comprising at least one phosphatidyloligoglycerol of Formula (VI")

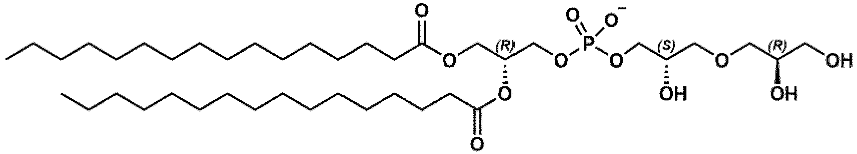

17. The liposome according to any one of claims 10 - 16, **characterized in that** it is a thermosensitive liposome.

18. The liposome according to any one of claims 10 - 17, further comprising an active agent, in particular doxorubicin
